# EUROPÄISCHE PATENTANMELDUNG

(11) **EP 2 589 380 A2**
(43) Veröffentlichungstag der Anmeldung: **08.05.2013**
(21) Anmeldenummer: 13153309.3
(22) Anmeldetag: 29.05.2008
(51) Int. Cl.: A61K 9/70, D06M 11/83, D03D 15/00, D06M 15/61, D06M 15/63, D06M 10/10, A61N 1/30

(54) **Verfahren zur Herstellung eines elektrisch leitfähigen Garns, textile Struktur für einen transdermalen Wirkstoffspeicher und Verfahren zur Wirkstoffspeicherung**

(30) Priorität: 29.05.2007 DE 102007024820; 28.05.2008 DE 102008025525
(62) Teilanmeldung aus: 08157234.9
(71) Anmelder: Textilforschungsinstitut Thüringen-Vogtland e.V., 07973 Greiz (DE)
(72) Erfinder: Neudeck, Andreas, 08468 Reichenbach (DE); Möhring, Uwe, 07987 Mohlsdorf/Reudnitz (DE); Scheibner, Wolfgang, 08539 Schönberg (DE)
(74) Vertreter: Kruspig, Volkmar

(57) **Zusammenfassung**

Die Erfindung betrifft eine Vorrichtung zum Ausführen eines Verfahrens zum Speichern und elektrochemischen Freisetzen eines Wirkstoffes in einem transdermalen Wirkstoffreservoir mit einer Verwendung eines Wirkstoffspeichers aus einem zu einem Anionenaustausch neigenden Redoxpolymer und einem anionischen Wirkstoff, wobei diese eine Anordnung zur transdermalen Wirkstoffübertragung in Verbindung mit einer Anordnung zum Ausführen einer Iontophorese aufweist, umfassend einen Speicherabschnitt (130) mit einem aus dem Redoxpolymer bestehenden Wirkstoffspeicher und einem von dem Speicherabschnitt entfernt angeordneten iontophoretischen Abschnitt (132) im Kontakt mit einer Epidermis in Verbindung mit einer mit variablen Potentialen beaufschlagbaren Anschlussanordnung (133) aus einem ersten Anschluss (134) zur Kontaktierung der Gegenelektrode des Speicherabschnittes, einem zweiten Anschluss (135) zur Kontaktierung einer mit dem Redoxpolymer beladenen Redoxpolymerelektrode des Speicherabschnittes und einem Anschluss (136) einer Elektrode des iontophoretischen Abschnittes.

## Beschreibung

Die Erfindung betrifft eine Vorrichtung zum Ausführen eines Verfahrens zum Speichern und elektrochemischen Freisetzen eines Wirkstoffes in einem transdermalen Wirkstoffreservoir nach Anspruch 1 - 3 sowie die Verwendung einer solchen Vorrichtung nach Anspruch 4.

Transdermale therapeutische Systeme, im folgenden als TTS bezeichnet, sind seit Beginn der 1980er Jahre bekannt und werden heute mit einer Reihe von Wirkstoffen in großem Maße eingesetzt. Mit diesen Systemen, die optisch herkömmlichen Pflastern vergleichbar sind, werden Wirkstoffe (wie z.B. Hormone, Nikotin u.a.) durch die Haut, d.h. transdermal, verabreicht. Die TTS stellen eine der wichtigsten pharmazeutisch technologischen Innovationen der letzten Jahre dar. Der Vorteil dieser Darreichungsform besteht in dem Übergang des Wirkstoffs in die Blutgefäße unter Umgehung des Magen-Darm-Traktes. Dieser wird geschont und eine Verstoffwechslung des Wirkstoffes bei der Resorption und durch die Leberfunktion vor Erreichen des Wirkortes wird reduziert. Weiterhin wird die Patienten - Akzeptanz (Compliance) verbessert, weil die Applikation der TTS nicht täglich, sondern in größeren Zeitabständen erfolgen kann. Grundsätzlich zeichnen sich die derzeit bekannten TTS durch folgende Merkmale aus:

Es erfolgt eine definierte Wirkstoffabgabe an die Blutgefäße der Haut, dabei befindet sich eine Gesamtdosis des Wirkstoffes gespeichert im TTS, die innerhalb einer gewissen Zeit in die Haut abgegeben wird. Ein TTS umfasst eine Gesamtfläche und eine sich in ihrer Größe eventuell davon unterscheidende Fläche für die Wirkstoffabgabe. Zusätzlich ist eine für den Wirkstoff undurchlässige Abdeckfolie vorgesehen, die auch als backing layer bezeichnet wird. Der Wirkstoff ist in einem Wirkstoffvorrat gespeichert. Dieser ist entweder in Form eines Behältnisses oder Reservoirs, in dem der Wirkstoff beispielsweise in Form einer Lösung enthalten ist, oder einer Wirkstoffmatrix ausgebildet, bei der der Wirkstoff an einem Speicherstoff absorbiert ist und von dem Speicherstoff abgegeben wird. Weiterhin ist ein Kontrollelement vorhanden, das die Wirkstoffzufuhr zur Haut regelt. Dies kann beispielsweie eine Membran oder einbettende Matrix sein. Desweiteren ist eine Haftschicht vorhanden, die für eine stabile Befestigung des TTS auf der Haut sorgt. Für eine derartige Haftschicht werden oft so genannte pressure sensitive adhesives (PSA) verwendet. Schließlich ist eine abziehbare Schutzschicht als so genannte Releaseliner vorhanden, die unmittelbar vor der Applikation des TTS auf die Haut entfernt wird. Es können auch mehrere Funktionen von ein und demselben Element erfüllt werden.

Die TTS werden nach Art und Weise der Wirkstofffreisetzung klassifiziert. Bei einem TTS mit Membran-permeationskontrollierter Freisetzung wird auf eine Polymermembran zurückgegriffen, welche die Permeation des Wirkstoffes aus dem Reservoir in die Haut kontrolliert. Diese besteht beispielsweise aus einem PVA-VA-Copolymer. Die Polymermembran kann auf unterschiedliche Weise mit dem Reservoir verbunden sein. Die Verbindung kann beispielsweise durch ein Verkapseln, Mikroverkapseln und andere Verfahren realisiert werden. TTS mit diesem Wirkprinzip haben den Vorteil einer relativ konstanten Rate der Wirkstofffreisetzung. Nachteilig bei derartigen TTS ist jedoch, dass bei mechanischer Beschädigung der Wirkstoff schlagartig in einer hohen Einzeldosis freigesetzt werden kann. Eine Verwendung von TTS mit einem derartigen Prinzip ist daher in Verbindung mit hochaktiven Pharmaka mit einem hohen Risiko behaftet.

Bei einem TTS mit Matrix-diffusionskontrollierter Freisetzung ist kein separates Kontrollelement enthalten. Die Wirkstofffreisetzung wird durch eine lipophile oder hydrophile Polymermatrix und/oder die Haftschicht kontrolliert. Das Wirkstoffreservoir wird durch den in der Matrix gelösten Wirkstoff oder eine homogene Dispersion fester Arzneistoffpartikel gebildet. Dies geschieht durch Mischen der Arzneistoffpartikel mit einem viskosen oder halbfesten Polymer bei Raumtemperatur und nachfolgender Vernetzung der Polymerketten, durch Vermischen bei höherer Temperatur oder durch Lösen beider Komponenten in einem organischen Lösungsmittel und anschließender Vakuumverdampfung des Lösungsmittels. Derartige auch als Matrix-TTS bezeichnete Transdermale Systeme geben den Arzneistoff nicht immer zeitkonstant ab. Eine schlagartige Freigabe ist allerdings nicht möglich, so dass diese bei hochaktiven Pharmaka sicher angewandt werden können.

Bei TTS mit Mikroreservoir-lösungskontrollierter Freisetzung sind zahlreiche 10-200 µm große Mikrokompartimente, die den Wirkstoff enthalten, in die Matrix eingelagert. Diese bilden zugleich ein Reservoir und ein Abgabekontrollelement.

TTS mit stromkontrollierter Freigabe weisen eine Abdeckfolie mit elektronischem Zubehör, eine Trennschicht, eine Anode und eine Kathode auf. Unter dem Einflus eines elektrischen Feldes werden Wirkstoffstoffkationen, und negativ geladene Gegenionen bewegt, wobei die Wirkstoffkationen auf die Haut ausgeschleust werden. Diese TTS sind bisher noch nicht kommerziell verfügbar. Ein Prototyp für Peptide ist bekannt. In der Entwicklung sind Einmal-TTS, bei denen alle Komponenten in einem dünnen flexiblen Polymerfilm enthalten sind und Systeme zum mehrmaligen Gebrauch, bei denen die elektronische Kontrolleinheit beliebig oft mit frischen polymeren Wirkstoffträgern versehen werden kann.

TTS mit schallkontrollierter Freigabe stehen in der Entwicklung noch zurück, da ihr therapeutischer Wert noch nicht ausreichend bewiesen ist. Es ist erwiesen, dass durch Schall und insbesondere Ultraschall ein Wirkstoffdurchtritt durch die Haut bis auf das 20-fache des Durchtritts ohne Schallanwendung gesteigert werden kann.

Die Anwendung einer Iontophorese stellt eine weitere Möglichkeit zur gesteuerten Wirkstofffreisetzung dar.

Die TTS der vorhergehend genannten Ausführungsformen weisen jedoch einige gravierende Nachteile auf. Diese ergeben sich insbesondere durch die Wirkung der TTS am Applikationsort. So ist bekannt, dass TTS bei längerer Anwendung am unveränderten Applikationsort die Haut schädigen können. Je nach individuellen Hauttyp können Allergien zu Bestandteilen des TTS ausgebildet werden. Die Wirkung des TTS tritt infolge der erforderlichen Hautsättigung erst verspätet ein. Hinzu kommen noch Probleme durch teilweise ungenügende Haftung auf der Haut und Stabilitätsprobleme durch nachträgliches Kristallisieren des Wirkstoffes bei Matrixpflastern.

Es besteht die der Erfindung zugrunde liegende Aufgabe, eine für eine TTS verwendbare Vorrichtung zum Ausführen eines Verfahrens zum Speichern und elektrochemischen Freisetzen eines Wirkstoffes in einem transdermalen Wirkstoffreservoir anzugeben, mit dem eine zuverlässige Wirkstoffspeicherung bei einer genau einstellbaren Freisetzungsrate möglich ist. Das damit geschaffene TTS soll atmungsaktiv, platzsparend und allergenarm sein.

Die Lösung der Aufgabe erfolgt mit einer Vorrichtung zum Ausführen eines Verfahrens zum Speichern und elektrochemischen Freisetzen eines Wirkstoffes in einem transdermalen Wirkstoffreservoir mit den Merkmalen des Anspruchs 1 bis 3 und mit der Verwendung einer Vorrichtung mit den Merkmalen des Anspruches 4.

Die erfindungsgemäße Vorrichtung gemäß Anspruch 1 bis 3 weist eine Anordnung zur transdermalen Wirkstoffübertragung in Verbindung mit einer Anordnung zum Ausführen einer Iontophorese auf, umfassend einen Speicherabschnitt mit einem aus dem Redoxpolymer bestehen den Wirkstoffspeicher und einem von dem Speicherabschnitt entfernt angeordneten iontophoretischen Abschnitt im Kontakt mit einer Epidermis in Verbindung mit einer mit variablen Potentialen beaufschlagbaren Anschlussanordnung aus einem ersten Anschluss zur Kontaktierung der Gegenelektrode des Speicherabschnittes, einem zweiten Anschluss zur Kontaktierung einer mit dem Redoxpolymer beladenen Redoxpolymerelektrode des Speicherabschnittes und einem Anschluss einer Elektrode des iontophoretischen Abschnittes.

Ein Verfahren zum Speichern und elektrochemischen Freisetzen eines Wirkstoffes in einem transdermalen Wirkstoffreservoir zeichnet sich durch folgende Merkmale aus:

Es wird ein Wirkstoffspeicher aus einem zu einem Anionenaustauch neigenden Redoxpolymer und einem anionischen Wirkstoff mit folgenden Verfahrensschritten verwendet:

Zu Beginn erfolgt ein Reduzieren des Redoxpolymers zum Freisetzen unerwünschter, im Polymergerüst gebundener Gegenionen. Dieser Schritt ist sinnvoll, weil das Redoxpolymer unter Normbedingungen in der Regel im oxidierten Zustand vorliegt und daher Ionen aufgenommen hat, die aus dem Polymergerüst entfernt werden müssen.

Im Anschluss erfolgt ein Oxidieren des Redoxpolymers und eine Einlagerung des anionischen Wirkstoffes in die Speicherschicht in einem Speicherschritt. In einem späteren Freisetzungsschritt wird das Redoxpolymer reduziert und der anionische Wirkstoff aus der Speicherschicht freigesetzt.

Das Verfahren baut auf dem Gedanken auf, den Umstand auszunutzen, dass ein Redoxpolymer in Abhängigkeit von dessen Oxidationszustand Änderungen in seiner molekularen Konfiguration ausführt und dabei ein unterschiedliches Speichervermögen für Anionen in der Polymermatrix aufweist. Der zu speichernde Wirkstoff wird in einer anionischen Form in der Matrix des Redoxpolymers gespeichert, sofern das Redoxpolymer oxidiert ist. Bei einer Reduktion konfiguriert sich das Redoxpolymer um. Die gespeicherten Anionen, d.h. also der anionische Wirkstoff, werden nun aus der Polymermatrix herausgedrängt und damit freigesetzt. Es liegt also in Form des Redoxpolymers ein Wirkstoffspeicher für den betreffenden Wirkstoff vor, der geschaltet werden kann. Dabei ist insbesondere ein Schalten auf elektrochemischem Wege möglich.

Dies kann mit einem Abscheiden des Redoxpolymers auf einem leitfähigen textilen Träger ausgeführt werden. Hierzu werden folgende Verfahrensschritte ausgeführt:

Es erfolgt ein elektrochemisches Abscheiden des Redoxpolymers auf dem Träger im reduzierten Zustand. Das nun auf dem Träger befindliche Redoxpolymer wird in eine wässrige Lösung des anionischen Wirkstoffes eingebracht. Der Träger wird nun als Anode geschaltet. Dadurch werden positive Ladungsstrukturen im Polymergerüst des oxidierten Redoxpolymers erzeugt. Der anionische Wirkstoff wird dadurch aus der wässrigen Lösung in das Polymergerüst eingebaut. Abschließend wird der Träger mit dem nun mit dem Wirkstoff angereicherten Redoxpolymer aus der Lösung entfernt. Das Redoxpolymer ist nun mit dem Wirkstoff beladen und der Träger ist nun als Wirkstoffreservoir verwendbar.

Auch kann ein Wirkstoffspeicher aus einem zu einem Kationenaustausch neigenden Redoxpolymer verwendet werden. In diesem Fall besteht das Redoxpolymer aus einem intrinsisch leitfähigen Polymer mit inkorporierten unbeweglichen Anionen, insbesondere Toluensulfonat oder einem Polymersulfonat. Dabei werden folgende Verfahrensschritte ausgeführt:

Es erfolgt ein Reduzieren des Redoxpolymers in einer Lösung aus einem kationischen Wirkstoff und ein Einlagern des kationischen Wirkstoffes in den Wirkstoffspeicher in einem Speicherschritt. In einem Freisetzungsschritt erfolgt ein Oxidieren des Redoxpolymers und ein Freisetzen des kationischen Wirkstoffes aus dem Wirkstoffspeicher.

Auch kann ein Wirkstoffspeicher aus einem zu einem Kationenaustausch neigenden Redoxpolymer im Kontakt mit einer mit einem kationischen Wirkstoff beladenen Reservoir-Polymerschicht mit folgenden Verfahrensschritten verwendet werden:

Es wird eine Oxidation des Redoxpolymers und Speichern von Kationen in der Redoxpolymerschicht ausgeführt. Eine Reduktion des Redoxpolymers führt zu einem Freisetzen von Kationen aus dem Redoxpolymer in die Reservoir-Polymerschicht. In der Reservoir-Polymerschicht erfolgt dann ein Verdrängen des kationischen Wirkstoffes aus der Reservoir-Polymerschicht in einen angrenzenden Epidermisbereich, wobei der Wirkstoff dann durch die Haut aufgenommen werden kann.

Der Wirkstoffspeicher ist als ein Teil einer elektrisch schaltbaren textilen Struktur ausgebildbar. Dabei ist eine erste textile Elektrode mit dem Redoxpolymer modifiziert, während eine zweite textile Elektrode als Gegenelektrode ausgebildet ist, wobei sich zwischen der ersten und der zweiten textilen Elektrode eine ionenleitfähige Textilstruktur befindet. Dabei ist ein Redoxzustand des Redoxpolymers und die Freisetzung des Wirkstoffes und die Freisetzung des Wirkstoffes durch eine geeignete Polung der ersten und der zweiten textilen Elektrode einstellbar.

Die vorhergehend genannten Verfahrensschritte und Vorrichtungsmerkmale können insbesondere für eine Anordnung zur transdermalen Wirkstoffübertragung in Verbindung mit einer Anordnung zum Ausführen einer Iontophorese vorteilhaft angewendet werden. Die Anordnung enthält einen Speicherabschnitt mit einem aus dem Redoxpolymer bestehenden Wirkstoffspeicher und einen von dem Speicherabschnitt entfernt angeordneten iontophoretischen Abschnitt im Kontakt mit einer Epidermis in Verbindung mit einer mit variablen Potentialen beaufschlagbaren Anschlussanordnung. Die Anschlussanordnung besteht aus einem ersten Anschluss zur Kontaktierung der Gegenelektrode des Speicherabschnittes, einem zweiten Anschluss zur Kontaktierung einer mit dem Redoxpolymer beladenen Redoxpolymerelektrode des Speicherabschnittes und einem Anschluss einer Elektrode des iontophoretischen Abschnittes.

Eine derartige Anordnung kann vor allem in Hinblick auf den iontophoretischen Abschnitt vorteilhaft als eine aktuatorische und/oder sensorische Elektrode zur Übertragung körpereigener und/oder körperfremder elektrischer Signale ausgebildet sein.

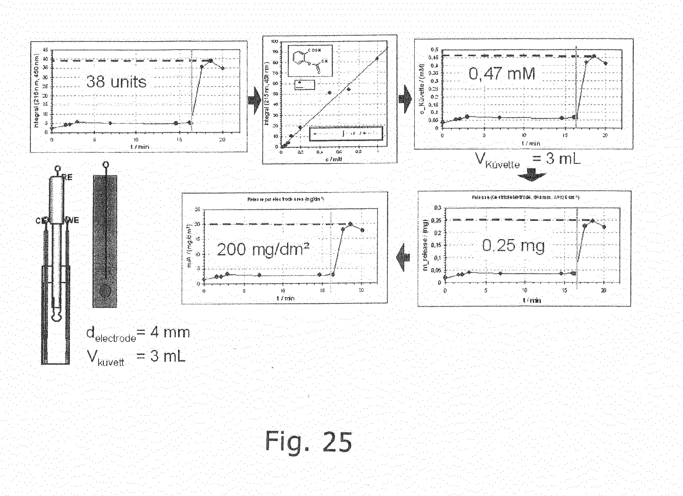

Die Erfindung soll nun nachfolgend anhand einiger Ausführungsbeispiele näher erläutert werden. Zur Verdeutlichung dienen die beigefügten Figuren 1 bis 39. Es zeigt:
- Fig. 1: elektrochemisch modifizierte Fadenelektroden für ein Wirkstoffspeichersystem in Form einer dreilagigen Gewebestruktur und in Form einer gewebten Interdigitalstruktur,
- Fig. 2: ein Prinzip eines elektrophoretischen Entfernens von Wasser vom Hautkontakt,
- Fig. 3: eine schematische Darstellung eines elektroosmotischen Feuchtetransports in einem elektrolytgetränkten Textilsubstrat,
- Fig. 4: einen schematischen Aufbau eines Messplatzes zum Bestimmen des Einflusses des elektrischen Feldes an einem Abstandsgewirke auf den Feuchtetransport,
- Fig. 5: eine schematische Darstellung des Messplatzes und des Feuchtigkeitsstroms durch das Abstandsgewirke nach Vorgabe einer definierten Wassermenge ohne angelegtes Potential,
- Fig. 6: den Messplatz aus Fig. 5 mit einem Potential, bei dem die Elektroosmose dem Feuchtigkeitsstrom entgegen wirkt,
- Fig. 7: den Messplatz aus Fig. 5 mit einem Potential, bei dem die Elektroosmose den Feuchtigkeitsstrom beschleunigt,
- Fig. 8: ein den Einfluss des elektrischen Feldes an einem Abstandsgewirke auf den Feuchtetransport illustrierendes Diagramm,
- Fig. 9: ein den Einfluss des elektrischen Feldes an einem Abstandsgewirke auf den Feuchtetransport illustrierendes Diagramm, Darstellung des Feuchtegehaltes in der unteren textilen Fläche,
- Fig. 10: eine Darstellung eines hydrolytischen Polymerabbaus und einer Wirkstofffreisetzung durch elektrochemisch induzierte pH-Änderung in einer wirkstoffbeladenen Polymerschicht,
- Fig. 11: eine schematische Darstellung eines Schichtaufbaus zum Einsatz eines Redoxpolymers als Wirkstoffspeicher- und Dosiersystem für einen anionischen Wirkstoff,
- Fig. 12: eine schematische Darstellung des Schichtaufbaus für den Einsatz eines Redoxpolymers als Wirkstoffspeicher- und Dosiersystem in Kontakt mit einer Lösung des anionischen Wirkstoffs ohne äußeres Potential,
- Fig. 13: eine schematische Darstellung des Schichtaufbaus für den Einsatz eines Redoxpolymers als Wirkstoffspeicher- und Dosiersystem in Kontakt mit einer Lösung des anionischen Wirkstoffs bei angelegtem äußeren Potential mit einer Oxidation des Polymers,
- Fig. 14: eine schematische Darstellung des Schichtaufbaus für den Einsatz eines Redoxpolymers als Wirkstoffspeicher- und Dosiersystem in Kontakt mit einer Lösung des anionischen Wirkstoffs bei angelegtem äußeren Potential mit einer Reduktion des Polymers zur Wirkstofffreisetzung,
- Fig. 15: eine beispielhafte Einlagerung von Acetylsalicylat als Gegenion in eine Struktur oxidierter Kettenabschnitte von Polyanilin,
- Fig. 16: eine beispielhafte Einlagerung von Acetylsalicylat als Gegenion in eine polaronisch und bipolaronische Molekülstruktur eines elektrochemisch oxidierten Kettenabschnittes von Poly-3,4-ethylendioythiophen (PEDOT),
- Fig. 17: eine Darstellung einlaminierter, mit Redoxpolymeren modifizierter Fadenelektroden,
- Fig. 18: ein CV einer mit dem Redoxpolymer Polyanilin modifizierten Fadenelektrode,
- Fig. 19: eine Darstellung der Dissoziationszustände von Acetylsalicylsäure mit ihrem Anion und ihrem Dianion und von Nikotinsäure mit ihrem Anion,
- Fig. 20: eine Darstellung der Konzentrationsabhängigkeit von UV-VIS-Spektren von Acetylsalicylsäure in 0,1 M NaCL-Lösung,
- Fig. 21: eine Darstellung der Konzentrationsabhängigkeit von UV-VIS-Spektren von Nikotinsäure in 0,1 M NaCI-Lösung,
- Fig. 22: eine schematische Darstellung einer Messzelle zum spektroelektrochemischen Nachweis einer elektrochemisch induzierten Wirkstofffreisetzung aus mit Redoxpolymeren modifizierten wirkstoffbeladenen textilen Elektroden,
- Fig. 23: UV-VIS-Spektren mit zugehörigen Extinktionzeitkurven bei einer Wellenlänge von 233 nm und 292 nm zum Nachweis der interaktiven Freisetzung von in Polyanilin gespeicherter Acetylsalicylsäure aus textilen Gestrickelektroden,
- Fig. 24: UV-VIS-Spektren mit zugehörigen Extinktionzeitkurven bei einer Wellenlänge von 233 nm und 292 nm zum Nachweis der interaktiven Freisetzung von in PEDOT gespeicherter Acetylsalicylsäure aus textilen Gestrickelektroden,
- Fig. 25: Diagramme zur Bestimmung einer Wirkstoffkapazität einer mit PEDOT präparierten Gestrickelektrode für Acetylsalicylsäure gemäß Fig. 24, aufgezeichnet während der Wirkstofffreisetzung,
- Fig. 26: UV-VIS-Spektren und zugehörige Extinktionszeitkurven bei einer Wellenlänge von 261 nm zum Nachweis einer Freisetzung von in Polyanilin gespeicherter Nikotinsäure aus textilen Gestrickelektroden,
- Fig. 27: UV-VIS-Spektren und zugehörige Extinktionszeitkurven bei einer Wellenlänge von 261 nm zum Nachweis einer Freisetzung von in PEDOT gespeicherter Nikotinsäure aus textilen Gestrickelektroden,
- Fig. 28: Diagramme zur Bestimmung einer Wirkstoffkapazität einer mit PEDOT präparierten Gestrickelektrode für Nikotinsäure zu den in Fig. 27 dargestellten UV-VIS-Spektren,
- Fig. 29: eine schematische Darstellung des Ablaufs einer Präparation von Rundgestricken mit Redoxpolymeren unter Verwendung von Polymerdispersionen zur Fertigung redoxpolymermodifizierter Garne,
- Fig. 30: eine illustrative Darstellung verschiedener Präparationsschritte an einem Garn,
- Fig. 31: eine schematische Darstellung der Technologie zum Beladen von mit Redoxpolymeren modifizierten Rundgestricken mit Wirkstoffen zur Fertigung von wirkstoffbeladenen Rundgestricken und Garnen,
- Fig. 32: eine schematische Darstellung einer gewebten Interdigitalstruktur mit einem wirkstoffbeladenen, redoxpolymermodifizierten Garn und einem hochleitfähigen Polyamidgarn ohne und mit nicht leitfähigen Kett- und Schußfäden,
- Fig. 33: eine schematische Darstellung des Aufbaus eines TTS aus zwei Drehergeweben, bei denen ein isolierender Multifilamentkettfaden eine erste und ein Schußfaden eine zweite Gewebeseite bildet, die durch einen Monofilamentfaden zusammengehalten werden und eine schematische Darstellung des Verlaufs der alternierend eingezogenen Kettfäden im Falle einer Leinwandbindung,
- Fig. 34: zwei Beispiele mehrlagiger Gewebestrukturen in fünflagiger und vierlagiger Ausführung,
- Fig. 35: eine beispielhafte Darstellung zum Nachweis einer Wirkstoffabgabe aus einer vierlagigen Gewebestruktur mit wirkstoffbeladener textiler Elektrode und Gegenelektrode,
- Fig. 36: eine schematische Darstellung eines Schichtaufbaus zum Einsatz eines Redoxpolymers als ein aktives System zur Ionenausschüttung im Kontakt mit einer mit einem ionischen Wirkstoff beladenen Polymerschicht als Wirkstoffspeicher- und Dosiersystem,
- Fig. 37: eine schematische Darstellung eines Schichtaufbaus für den Einsatz eines Redoxpolymers im reduzierten Zustand im Kontakt mit einer mit ionischem Wirkstoff beladenen Polymerschicht,
- Fig. 38: eine schematische Darstellung des Schichtaufbaus aus Fig. 37 mit dem Redoxpolymer im oxidierten Zustand,
- Fig. 39: eine schematische Darstellung des Aufbaus eines interaktiven textilen TTS mit gekoppelter Iontophorese im Zustand der Wirkstoffspeicherung im oxidierten Redoxpolymer, der Wirkstofffreisetzung durch Reduktion des Redoxpolymers und des iontophoretischen Transports des Wirkstoffes durch die Haut (Wirkstoffübertragung).

Es werden für gleiche bzw. gleichwirkende Teile und Verfahrensschritte dieselben Bezugszeichen verwendet.

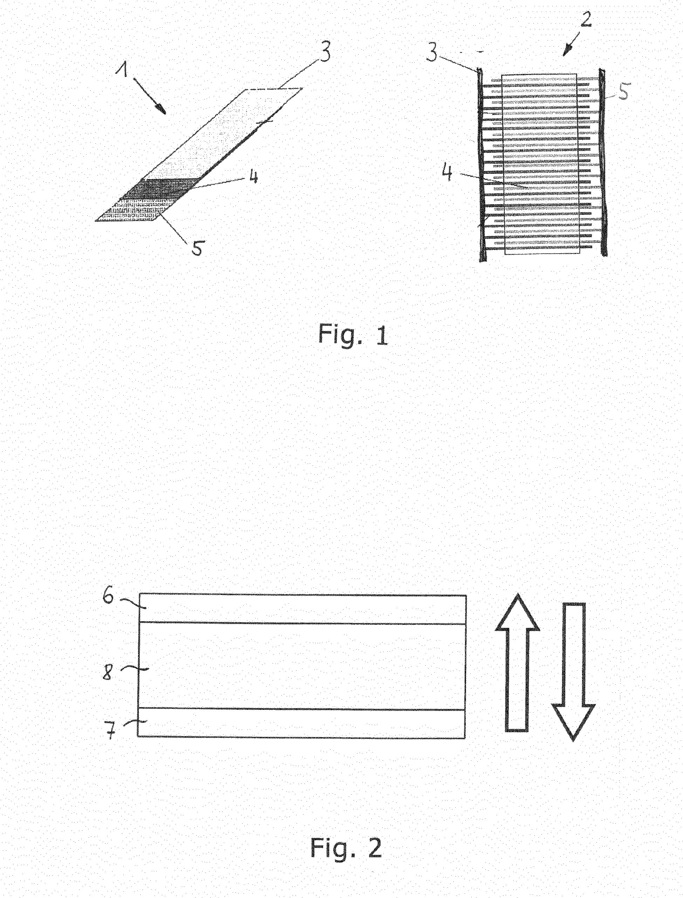

Fig. 1 zeigt in zwei verschiedenen Prinzipdarstellungen textiler Gewebe mit elektrochemisch modifizierten Fadenelektroden für ein Wirkstoffspeichersystem in Form einer dreilagigen Gewebestruktur 1 und in Form einer gewebten Interdigitalstruktur 2. Bei den folgenden Beschreibungen und Ausführungsformen wird von chemisch versilberten Polyamidfäden und daraus erzeugten textilen Strukturen ausgegangen, die sich galvanisch und elektrochemisch modifizieren lassen. Dies betrifft insbesondere partiell leitfähige mehrlagige Strukturen, wie sie für die Aufnahme, Kontaktierung und Verschaltung von mikroelektronischen Bauelementen benötigt werden, als auch Interdigitalstrukturen, die in der Sensorik und die erstmals zum Aufbau von textilen Lichtquellen eingesetzt werden. Beide in Fig. 1 gezeigte Strukturen sind für die TTS realisierbar. Derartige TTS werden im Folgenden auch als textile transdermale therapeutische Systeme, bzw. textile TTS oder auch ti-TTS bezeichnet.

Gemäß den Prinzipdarstellungen aus Fig. 1 bestehen die im folgenden beschriebenen Systeme aus metallisierten Fäden 3, die insbesondere eine Ag/AgCl-Bedeckung aufweisen, einem ionenleitenden Polymerfilament 4 und einem mit einem Redoxpolymer modifizierten Gewebebestandteil 5. Diese sind entweder übereinander geschichtet und bilden eine mehrlagige, insbesondere dreilagige, Gewebestruktur aus, deren Grundgerüst durch ein Garngewebe oder Gestricke gebildet wird, oder sie sind in Form gewebter Interdigitalstrukturen ausgeführt, bei der alle genannte Komponenten in einer einzigen Gewebefläche ineinander verwebt sind.

Im Folgenden werden beide Grundkonfigurationen sowie zusätzliche Konfigurationen anhand einiger Ausführungsbeispiele näher erläutert. Sowohl in der einen als auch in der anderen Form kann ein Mikrodosiersystem für einen Wirkstoff direkt in der textilen Struktur, auf der sich gleichzeitig der Schichtaufbau des TTS befindet, zusammen mit den benötigten Sensoren integriert oder selbst als textile Struktur ausgeführt sein.

In Verbindung damit ist es ebenfalls möglich, dass beispielsweise der pH-Wert der textilen Struktur kontrolliert und gezielt beeinflusst und der Wirkstoff in einer hydrolyseemfindlichen nanoporösen Polymermatrix auf der gleichen textilen Struktur freigesetzt werden kann.

Insgesamt werden bei den folgenden Ausführungsform damit folgende Lösungsansätze verfolgt: ein Elektrophoretisches Entfernen von Feuchtigkeit vom Hautkontakt, eine Hydrolytische Spaltung einer Wirkstoffträgermatrix durch elektrochemisch induzierte pH-Änderung, Redoxpolymere als Wirkstoffspeicher- und -spendersysteme und Redoxpolymere als ein Ionendosiersystem, bei dem aus wirkstoffhaltigen Polymermatrices anionische und kationische Wirkstoffe freigesetzt werden.

Nanoporöse wirkstoffhaltige Polymermatrices sind beispielsweise in Form von Hydrogelen verfügbar. Diese lassen sich auf ein textiles Substrat aufbringen. Wird ein Textil mit einer solchen Polymermatrix modifiziert und mit fadenförmigen Elektroden kombiniert, kann die wirkstoffhaltige Polymermatrix gezielt beeinflusst werden. Durch das Anlegen einer äußeren Spannung an die in das textile System, bestehend aus der nanoporösen wirkstoffhaltigen Polymermatrix und den fadenförmigen Elektroden kann sowohl der Feuchtegehalt, als auch der pH-Wert in der auf der textilen Struktur aufgebrachten Polymermatrix aktiv verändert werden.

Zunächst sollen Möglichkeiten einer gezielten Steuerung des Feuchtegehaltes von textilen Strukturen dargestellt werden. In geeigneten textilen Strukturen aus leitfähigen und nichtleitfähigen Fadenmaterialien kann die Feuchtigkeit in dem textilen Schichtsystem durch Anlegen einer Spannung an das integrierte textile Elektrodensystem durch Elektroosmose von der Innenseite in die äußeren Schichten oder aber aus Bereichen im Zentrum in die Randbereiche und umgekehrt transportiert werden. Dies ist in Fig. 2 angedeutet.

Fig. 2 zeigt einen beispielhaften Schichtaufbau in einem dreilagigen Gewebe mit einer ersten Fadenelektrode 6 und einer zweiten Fadenelektrode 7. Diese bestehen beispielsweise aus Polyamid und weisen eine Metallisierung aus Platin bzw. Silber auf. Zwischen den Fadenelektroden ist eine Polymermatrix 8 als ein nanoporöses Gel-System mit einem darin gespeicherten Wirkstoff angeordnet. Rechts neben der Schichtdarstellung sind durch Pfeile die Richtungen eines möglichen Feuchtetransports angedeutet. Eine Freigabe des Wirkstoffes in Richtung Haut erfordert einen Feuchtetransport von der oberen zur unteren Fadenelektrode. Bei einem entgegengesetzten Feuchtetransport ist die Freisetzung des Wirkstoffes aus dem Gel-System blockiert. Durch diesen als Elektrophorese bezeichneten Vorgang wird somit das Wasser, welches für die Übertragung des Wirkstoffes von der Polymermatrix auf die Haut notwendig ist, aus der Matrix entfernt bzw. für die Übertragung des Wirkstoffes von der Haut wieder in das Gel-System eingetragen.

Durch die gezielte Steuerung des Feuchtegehaltes der im Hautkontakt stehenden Schicht des textilen TTS kann somit die Geschwindigkeit des Wirkstofftransfers, der nur aus der feuchten nanoporösen Schicht erfolgt, gezielt beeinflusst werden. Die nanoporösen wirkstoffhaltige Polymermatrices sind so aufgebaut, dass der Wirkstoff durch einen hydrolytischen Abbau der Polymermatrix freigesetzt wird. Folglich kann die Geschwindigkeit der Wirkstoffabgabe durch den Feuchtegehalt und den pH-Wert gesteuert werden. Mit textilen Strukturen aus leitfähigen und nichtleitfähigen Fadenmaterialien kann zunächst der Feuchtegehalt in der Zone, in der die Polymermatrix vorhanden ist, über einen weiten Bereich gezielt reguliert werden.

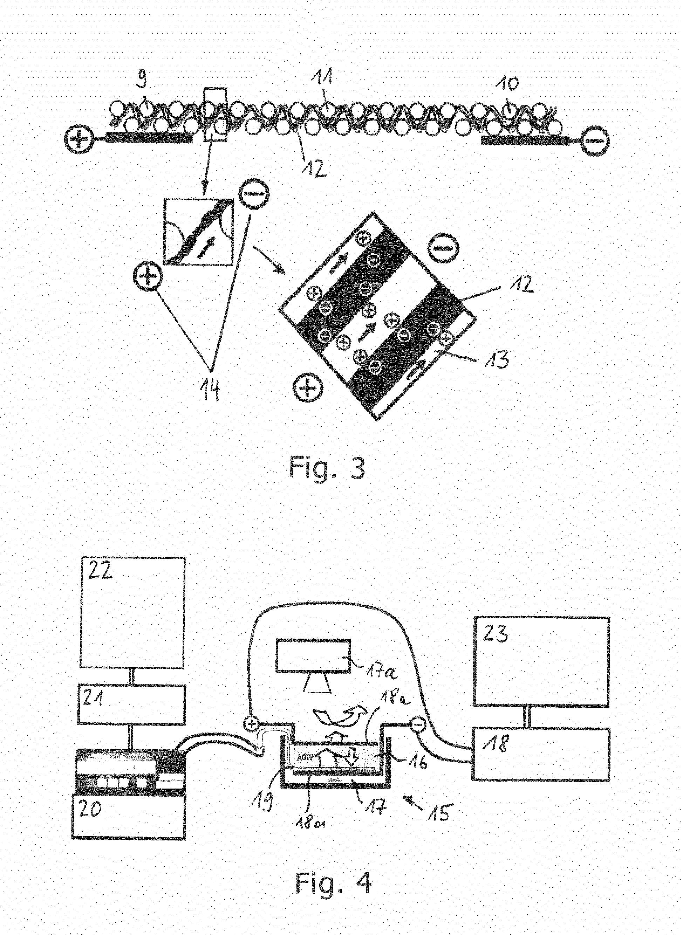

Eine derartige Elektroosmose kann in einem in Fig. 3 gezeigten Textilsubstrat ausgeführt werden. Das Textilsubstrat weist textile Anodenfäden 9 und textile Katodenfäden 10 als Kett- oder Schußfäden auf. Parallel dazu verlaufen eine Reihe von nicht leitenden Fäden 11. Der Feuchtetransport erfolgt durch ein Multifilament 12 in einem kapillaren Elektrolytfilm 13, in dem das zwischen den Anoden- und Katodenfäden anliegende elektrische Feld 14 wirkt. In diesem Grenzflächensystem findet eine Ladungstrennung statt. Das Filamentmaterial ist dabei negativ geladen, während sich im angrenzenden Elektrolytfilm positive Grenzflächenladungen ausbilden, die im Feld transportiert werden.

Bei der Elektroosmose wird an ein mit einem Elektrolyten gefülltes Kapillarsystem über Elektroden an beiden Seiten ein äußeres elektrisches Feld angelegt. Durch die Wirkung des äußeren elektrischen Feldes auf die sich im Kapillarsystem an der Grenzfläche Kapillarwand/Elektrolyt ausbildende diffuse elektrische Doppelschicht wird ein Stofftransport im Elektrolyten induziert. Es handelt sich um physiko-chemische Interaktionen zwischen Porenflüssigkeit und Feststoff in der Nähe der Kapillarwände. Die Richtung des Flüssigkeitsstroms folgt der Coehn`schen Regel, d.h. die Phase mit der höheren Dielektrizitätszahl (DEZ) lädt sich gegenüber der anderen positiv auf. Das Wasser in den Poren, als schwacher Elektrolyt (z.B. Körperschweiß), weist eine hohe DEZ auf; es strömt somit in Richtung der negativen Elektrode, der Kathode gemäß Fig. 3. In Fig. 3 wird das Kapillarsystem bereits durch ein textiles mit Elektrolyt (Körperschweiß) getränktes Textilsubstrat gebildet.

Aus dem Wirkprinzip der Elektroosmose wird bereits deutlich, dass eine der beiden Elektroden, mit denen das elektrische Feld im Elektrolyten erzeugt wird, als Anode polarisiert wird. Das an dieser Elektrode anliegende Potential ist abhängig von der zu erzeugenden Feldstärke, mit der der Feuchtetransport induziert wird. Dieses Potential kann entsprechend seiner Größe und dem verwendeten Elektrodenmaterial zu einer Korrosion des Materials führen. Um diese Korrosion zu verhindern, muss das Material an der Anodenseite sorgsam ausgewählt werden. Neben teuren inerten Materialien wie Gold und Platin, die auf Grund ihres Preise lediglich als dünne Schichten auf den Elektrodenkontakten eingesetzt werden können, kommen auch passivierbare Materialien, insbesondere Stähle, Carbonmaterialien (Graphite, Kohlenstofffasern, Carbonvliesse, karbonisierte Metallelektroden bis hin zu mit dotiertem Diamantschichten überzogene Substrate) wegen ihrer Korrosionsstabilität bei gleichzeitig vertretbaren Materialpreisen in Betracht. Als textile Anodenmaterialien bieten sich folglich Carbonvliesse, Carbonfasern, Stahlfasergarne, aber auch metallisierte Garne an, wenn diese mit Gold oder Platinschichten mit Schichtdicken von 50 nm bis 150 nm beauflagt werden. Vom Blickwinkel der textilen Verarbeitbarkeit und ihrer elektrischen Leitfähigkeit sind die metallisierten Garne den anderen Materialien häufig weit überlegen. Da diese jedoch lediglich vergoldet oder platiniert als Anodenmaterial zum Einsatz gelangen können, übertreffen sie diese auch deutlich im Preis-Leistungs-Verhältnis, so dass die Carbonmaterialien bezüglich ihrer Eigenschaften und preislich eine sehr gute Alternative darstellen.

Unter Raumluftbedingungen liegt der Flächenwiderstand R_{F} von Textilien unter Raumluftbedingungen im Bereich von einigen Mega- bis Gigaohm. Das entspricht einem spezifischen Widerstand der textilen Materialien von 10 kΩcm bis 20 MΩcm und Leitfähigkeiten von 1·10⁻⁴ Scm⁻¹ bis 1·10⁻⁸ Scm⁻¹. Insbesondere bei Polymerfasern hängt die Leitfähigkeit stark vom Restfeuchtegehalt und dem in der Restfeuchte vorhandenen Elektrolytgehalt ab. Geht man von einem PES-Gewebe 110 dtex/f30 mit 30 Filamenten mit einem Filamentdurchmesser von 25 µm und einer Flächenmasse von 100 gm⁻² aus mit einem für PES üblichen Feuchtegehalt von 0,8% und einem NaCl-Gehalt von 0,1 M (vergleichbar dem Hautschweiß mit einer Leitfähigkeit von 10 µS cm⁻¹), so kann man die Flächenleitfähigkeit des Gewebes aus diesen Parametern über ein einfaches Modell berechnen. Dazu wird angenommen, dass der aus der Restfeuchte berechnete Elektrolytfilm die Filamentoberflächen gleichmäßig überzieht und nur die Filamente der Kett- oder der Schussfäden zur Leitfähigkeit in Messrichtung beitragen. Für ein solches PES-Modellgewebe erhält man einen Flächenwiderstand von 5 MΩ/dm². Bei einer quadratischen Probe, bei der die Länge der Kontaktelektroden gleich deren gegenseitigem Abstand ist, weist beispielsweise eine 10 cm x 10 cm große Probe eines PES-Gewebes einen Widerstand von 5 MΩ auf. Bei einer angelegten Spannung von 24 V fließt somit ein Strom von 4,8 pA. Erhöht sich der Elektrolytgehalt im Gewebe durch Schweiß mit einer Salzkonzentration von 0,1 M NaCl auf bis zu 30%, verringert sich der Widerstand auf 130 kΩ und die Stromstärke steigt auf 185 pA.

In Extremfällen, zum Beispiel bei Schuhwerk, können durchaus Feuchtegehalte von 70% und mehr auftreten, was zu einem Stromfluss von 0,5 mA - 1mA bei vergleichbaren Anschlusselektrodenlängen und Abständen und einer angelegten Spannung von 24 V führt. Bei Batteriekapazitäten von gängigen Batterien von ca. 500 mAh könnte ein solches textiles Gewebe über eine Dauer von 500 h und mehr betrieben werden.

Flächenwiderstandsmessungen in Abhängigkeit vom Elektrolytgehalt (0,1 M NaCl) in vergleichbaren Polyestergeweben und -gewirken zeigen jedoch, dass die Flächenwiderstände deutlich höher liegen. So sinkt der Flächenwiderstand in einer textilen PES-Fläche mit einer Flächenmasse von 90 g m⁻² erst nach einem Elektrolytgehalt (0,1 M NaCl) von mehr als 10 % unter 3 MΩ/dm².

Die Effektivität des Feuchtetransports über Elektroosmose kann wie folgt abgeschätzt werden: Geht man von der gleichen Modellfläche bei gleicher angelegter Spannung aus und nimmt die osmotische Mobilität von Sandstein µₑₒ = 10⁻⁹ m²/Vs auch für das Textil an, dann ist für eine quadratische Textilfläche mit einer Seitenlänge von 10 cm eine Schichtdicke von 0,6 cm notwendig, um einen Feuchtetransport von 0,52 ml/h lateral aus der 1 dm² großen Fläche zu realisieren. Dies entspricht einer durchschnittlichen Verdunstungsrate eines Menschen von etwa 100 ml/h über eine Körperoberfläche von ca. 2 m². Es müssen somit kontinuierlich 0,5 ml von einer 10 cm x 10 cm großen Fläche pro Stunde lateral transportiert werden um den Feuchtegehalt der textilen Fläche definiert konstant zu halten. Dies scheint mit der beschriebenen eletroosmotischen Modellstruktur möglich.

Für die interaktive Feuchteregulation in einem textilen transdermalen terapeutischen System ist kein lateraler Feuchtetransport nötig, wenn Abstandstextilien eingesetzt werden. Abstandstextilien, insbesondere Abstandsgewirke, bestehen aus zwei textilen Flächen, die über Polfäden auf einem definierten Abstand gehalten werden. Eine der textilen Flächen kann so als Träger der wirkstoffbeladenen Polymermatrix dienen, die im Hautkontakt steht. Dabei stellt die Polfadenschicht das für die Elektroosmose benötigte Kapillarsystem dar, während die zweite textile Fläche als Feuchtespeicher ausgeführt sein muss, in die überschüssige Feuchte abtransportiert und aus der für die Wirkstofffreisetzung benötigte Feuchte für die hydrolytische Spaltung der wirkstoffbeladenen Polymermatrix entnommen werden kann. Da der interaktive Feuchtetransport zu diesem Zweck in beiden Richtungen induziert werden muss, müssen beide textilen Flächen als Anode geschaltet werden können. Am einfachsten kann dies mit auf die Abstandstextilien aufkaschierten Carbonvliesen realisiert werden. Die Effektivität des elektroosmotischen Feuchtetransportes hängt aber in entscheidendem Maß vom Aufbau des Kapillarsystems, also von der Konstruktion der Polfadenschicht ab.

Zur Untersuchung des elektroosmotisch stimulierten Feuchtetransportes kann eine in Fig. 4 gezeigte Messanordnung dienen, mit der über einen resitiven textilen Feuchtesensor der Feuchtegehalt in einer textilen Struktur, der lokale Feuchtegehalt in Abhängigkeit der Zeit und vom an die textile Struktur angelegten elektrischen Feld gemessen werden kann. Die Messanordnung enthält eine Messzelle 15 mit einem darin befindlichen kontaktierten Abstandstexil 16, unter dem sich eine Flüssigkeitsmenge 17 mit einer bestimmten Elektrolytkonzentration befindet. Ein Gebläse 17a ermöglicht ein Einstellen eines kontrollierbaren Luftstroms über der Probe zur Regulierung der Verdunstungsrate über dem Textil. Das Abstandstextil wird über eine Gleichspannungsquelle 18 und Elektroden 18a mit einer Gleichspannung beaufschlagt, deren Polung umgeschaltet werden kann. Die Elektroden können als Carbonvlieselektroden ausgebildet sein. Innerhalb des Abstandstextils ist ein Leitfähigkeitssensor 19 angeordnet, der mit einem Leitfähigkeitsmessgerät 20 gekoppelt ist. Über eine Schnittstelle 21 können die gemessenen Leitfähigkeitswerte an einen PC 22 ausgegeben und dort aufgezeichnet bzw. verarbeitet und ausgewertet werden. Der Leitfähigkeitssensor fungiert als Feuchtesensor, die Leitfähigkeitsmessung ermöglicht somit eine Bestimmung der Feuchte in der Umgebung des Sensors.

Dabei ist darauf zu achten, dass die Leitfähigkeitsmessung nicht durch das angelegte äußere elektrische Feld gestört wird. Hierzu ist ein Trenntrafo 23 vorgesehen, der mit der Gleichspannungsquelle 18 gekoppelt ist. Dieser dient einer galvanischen Entkopplung der regelbaren Gleichstromquelle für das applizierte elektrische Feld.

Zur Leitfähigkeitsmessung wird das Leitfähigkeitsmessgerät 20 als ein mit Wechselspannung arbeitendes 4-Punkt Leitfähigkeitsmessgerät betrieben. Dies erlaubt eine reproduzierbare Messung ohne Drift in der Basislinie in einem abgeschlossenen System konstanter Feuchte.

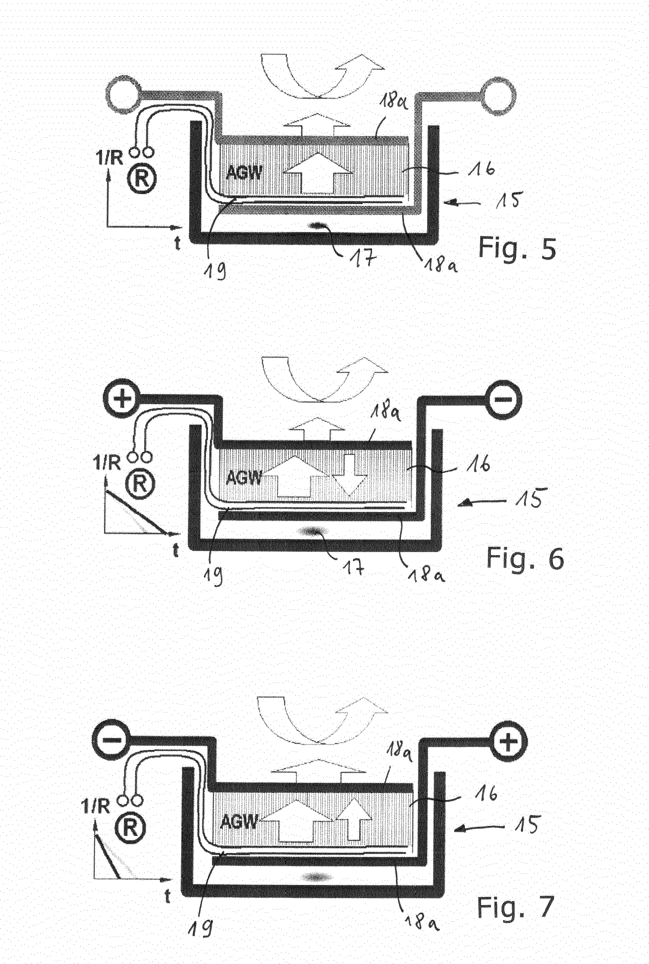

Dieser Aufbau ermöglicht das messtechnische Erfassen des in Fig. 5, Fig. 6 und Fig. 7 schematisch dargestellten Einflusses des angelegten elektrischen Feldes auf den Feuchtetransport.

Zur Untersuchung des durch das angelegte elektrische Feld beeinflussten Feuchtetransports an Abstandsgewirkestrukturen können die Abstandsgewirke beidseitig mit Carbonvliesselektroden ausgerüstet werden. Unter der unteren Carbonvliesselektrode ist zusätzlich der Feuchtesensor angebracht. Als textiler Feuchtesensor kommt eine gewebte Interdigitalstruktur zum Einsatz.

Die Elektroden des Feuchtesensors bestehen beispielsweise aus galvanisch nachversilberten Polyamidmonofilamenten mit einer Basisfeinheit von 22 dtex/f1. Der Durchmesser eines Monofilamentes inklusive der abgeschiedenen Silberschicht beträgt 60 µm. Durch den Doppelschusseintrag auf einer Nadelbandwebmaschine mit einer Schussdichte von 41 cm⁻¹ und den Verzicht auf einen Isolationsfaden zwischen den links und rechts am Gewebeband kontaktierten Kammelektroden resultiert ein Schusseintagsabstand der metallisierten Schüsse von 244 µm und folglich ein minimaler Abstand der leitfähigen Monofilamente von 124 µm. Die aus mikroskopischen Aufnahmen ermittelten Abstände stimmen sehr gut mit den theoretischen Abständen überein. Durch Anlöten von Drähten an den Kontaktfäden der gewebten Interdigitalstruktur links und rechts und ein Versiegeln dieser mit einer Silikonbeschichtung entsteht ein resistiver Feuchtesensor, der an textilen Substraten problemlos angebracht und in kaschierte Systeme integriert werden kann. Der Einfluss des Sensors auf den Feuchtetransport in der zu untersuchenden textilen Struktur kann vernachlässigt werden. Wenn der Sensor durch ein auf einer Wechselstrommessung basierenden Widerstands- bzw. Leitfähigkeitsmessgerät ausgelesen wird, werden keine Driften in der Basislinie beobachtet und man erhält reproduzierbare Widerstands- bzw. Leitwerte. Der gemessene Leitwert liefert, wie Kalibrationsmessungen zeigen, ein zum Feuchtegehalt proportionales Signal.

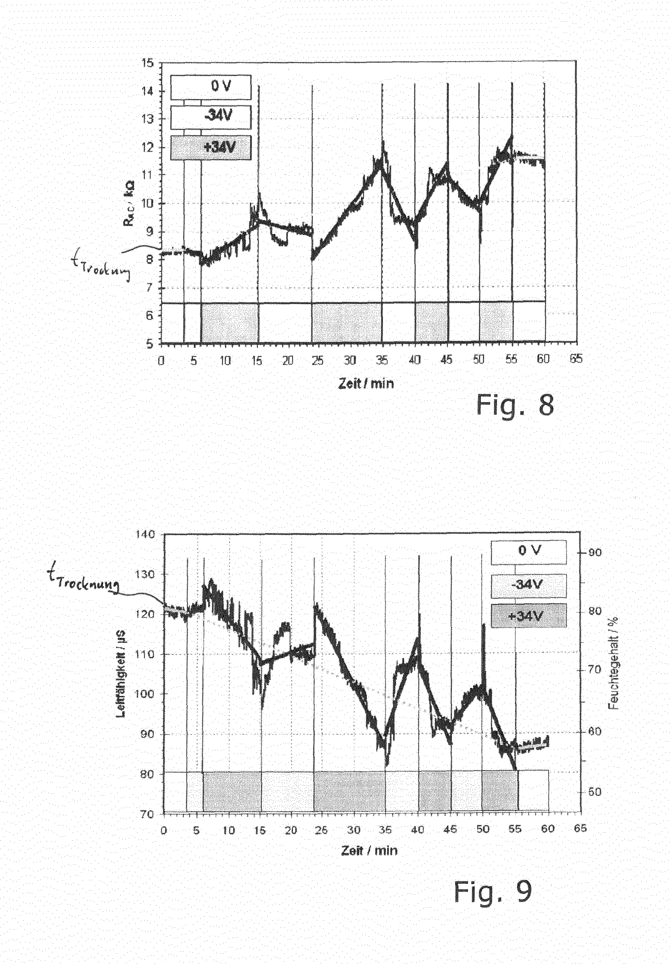

Die so präparierten Abstandsgewirke werden in eine Schale mit einer definiert vorgegebenen Wassermenge, z.B. 10 - 500 µl, gelegt, die Carbonvliesselektroden werden mit der Gleichspannungsquelle und die Sensoranschlüsse mit dem Leitfähigkeitsmessgerät verbunden. Anschließend beginnt die Aufzeichnung einer Leitwert-Zeit-Kurve ohne angelegtes äußeres elektrisches Feld. In definierten Zeitintervallen wird anschließend das elektrische Feld angelegt und alternierend die Polarität gewechselt. Eine so erfasste Messkurve ist in Fig. 8 beispielhaft dargestellt. Um den Einfluss des elektrischen Feldes auf den Feuchtetransport, der sich aus der Änderung der Restfeucht am Sensor in Abhängigkeit von der Zeit zu ermitteln, wird eine auf den Feuchtegehalt umgerechnete Messkurve erstellt. Diese ist beispielhaft in Fig. 9 gezeigt. Sie wird abschnittsweise durch eine Regressionsgerade interpoliert. Jeder Abschnitt der Messkurve entspricht entweder einem Zeitabschnitt ohne oder mit einem entsprechend der jeweiligen Polarität angelegten elektrischen Feld. Der Anstieg der Geraden ohne angelegtes äußeres elektrisches Feld ist gering und schwankt im Rahmen des Messfehlers zwischen positiven und negativen Werten, insbesondere aus dem ersten und letzten Zeitabschnitt der Messkurve in Fig. 9 zu ersehen ist.

Bestimmt man den Anstieg über einen längeren Zeitabschnitt ist der Anstieg leicht positiv. Dies resultiert aus dem verdunstenden Wasser pro Zeiteinheit an der Oberfläche des Abstandsgewirkes. Für eine quantitative Analyse der Feuchtetransportes ohne angelegtes elektrisches Feld in Abhängigkeit von der Struktur des Abstandsgewirkes sind mit einem solchen Versuchsaufbau zeitaufwendige Untersuchungen notwendig. In den Zeitintervallen mit angelegtem elektrischen Feld erhält man ausnahmslos positive Anstiege, wenn das obere Carbonvliess als Kathode und negative Anstiege, wenn die untere Carbonvliessschicht sls Kathode agiert. Der elektroosmotisch generierte Feuchtestrom ist folglich erwartungsgemäß zur Kathode hin gerichtet.

Allerdings ist dieser Effekt erst mit relativ hohen Feldstärken von mehr als 25 V/cm nachweisbar. Selbst bei dieser Feldstärke ist, wie in Fig. 9 ersichtlich, der Effekt zwar eindeutig messbar, für eine exakte quantitative Bestimmung ist das Signal-Rausch-Verhältins noch zu hoch. Deutlich bessere Ergebnisse können erzielt werden, wenn das Abstandsgewirke genau in das Messgefäß eingepasst wird, der textile Feuchtesensor sich über die gesamte untere Abstandsgewirkefläche erstreckt und die Anschlüsse zu den Carbonvliessen isoliert herausgeführt werden, wobei die Messzelle in einem Faradayschen Käfig untergebracht wird.

Eine Verkürzung der Messzeiten ist durch eine Beschleunigung des Feuchtetransportes durch eine definierte Luftströmung an der Oberseite des Abstandsgewirke und Verwendung des Gebläses erreichbar. Die Zeit t_{Trocknung} bis zum vollständigen Austrocknen der Struktur kann, wie in Fig. 8 gezeigt, aus dem Schnittpunkt der Regressionsgraden mit der Ordinate exakt bestimmt werden. Über die Beeinflussung und Bestimmung der Trocknungszeit kann folglich der Einfluss der Struktur des Abstandsgewirkes, der Messanordnung und des elektrischen Feldes systematisch untersucht werden.

Auch diese Untersuchungen bestätigen, dass bei Elektrolytgehalten deutlich unter 0,1 M hohe elektrische Feldstärken von mehr als 25 V/cm nötig sind, um merkliche Effekte zu erzielen. Setzt man an Stelle von Wasser 0,1 M Natriumchloridlösung, als eine Elektrolytkonzentration wie sie in etwa dem Hautschweiß entspricht, ein, so kann ein zusätzlicher, der Polarität des elektrischen Feldes entsprechender Feuchtigkeitsstrom bereits bei Feldstärken unter 10 V/cm nachgewiesen werden. Setzt man der vorgelegten Elektrolytmenge einen Säure-Base-Indikator (z.B. Phenolphthalein) zu, und betrachtet die als Kathode geschaltene Oberseite des Abstandsgewirkes bei einer Feldstärke, bei dem der elektroosmotische induzierte Feuchtetransport noch nachweisbar ist, so beobachtet man eine deutliche Rotfärbung. Die Rotfärbung zeigt an, dass der pH-Wert an der Kathode deutlich auf Werte größer 8,5 ansteigt. Ein solcher Anstieg wird durch die bereits an Anode und Kathode einsetzende elektrochemische Wasserzersetzung hervorgerufen. Durch die bereits einsetzende Wasserzersetzung steigt der pH-Wert durch die einsetzende Wasserstoffentwicklung stark an und sinkt entsprechend an der Anode durch die Sauerstoffentwicklung stark ab. Neben dieser drastischen Eigenschaftsänderung des Feuchtemilieus in der textilen Struktur wirkt sich die einsetzende Wasserzersetzung zusätzlich auf den Feuchtetransport aus. Eine Separation beider Prozesse ist nicht möglich, wenn der durch die angelegte Spannung erzeugte Feuchtestrom dem durch das Verdampfen an der Oberfläche erzeugten Feuchtestrom wirksam entgegenwirken bzw. beschleunigt werden soll.

Um die Hydrolyse einer mit Wirkstoffen beladenen Schicht, die sowohl vom pH-Wert als auch vom Feuchtegehalt abhängt, über den Feuchtegehalt zu steuern, muss dieser unabhängig vom pH-Wert einstellbar sein. Da dies nur mit hohem Aufwand realisiert werden kann, ist die elektroosmotisch über den Feuchtegehalt gesteuerte Wirkstofffreisetzung aus textilen Strukturen nur schlecht ausführbar.

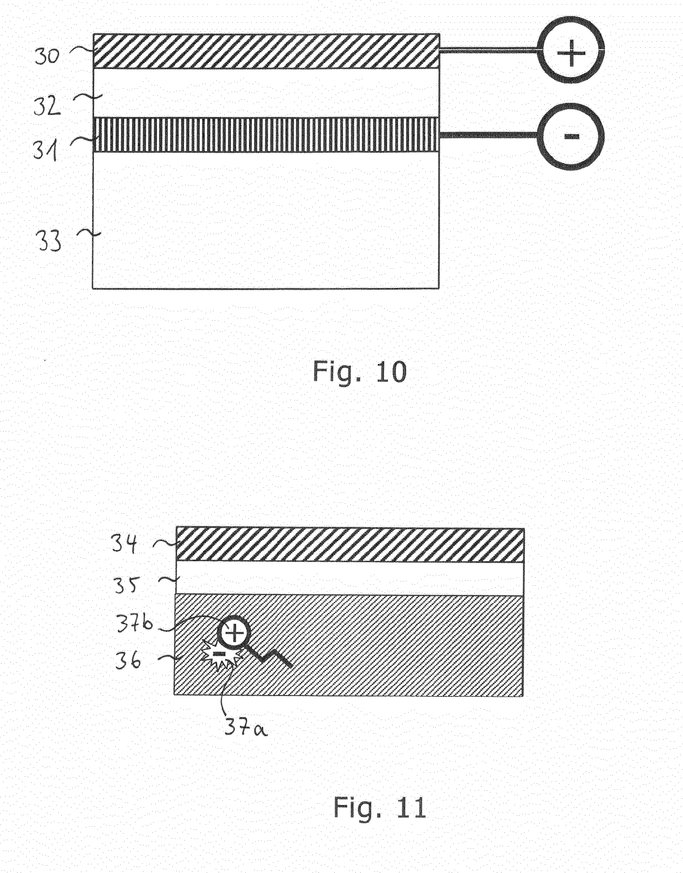

Wie im Folgenden in Verbindung mit Fig. 10 erläutert wird, kann durch eine geeignete Wahl der Gel- oder Polymerelektrolyte mit geringfügig höheren Spannungen als bei Elektroosmose auch der pH-Wert um die textilen integrierten fadenförmigen Elektroden und somit auch in der angrenzenden mit Wirkstoff beladenen Schicht verändert werden. Kommt in der Schicht die hydrolyseemfindliche, nanoporöse, mit Wirkstoff beladene Polymermatrix zum Einsatz, kann durch die angelegte Spannung zusätzlich der pH-Wert der Schicht beeinflusst und durch am Faden elektrochemisch erzeugte pH-sensitive Elektroden kontrolliert werden. Mit der pH-Kontrolle kann der hydrolytische Abbau der Polymermatrix und damit die Wirkstofffreisetzung gesteuert bzw. reguliert werden.

Gemäß der beispielhaften schematischen Darstellung in Fig. 10 ist dazu ein dreilagiges Gewebe realisiert. Dieses besteht aus einer Platin/Silber/Polyamid-Fadenelektrode 30 mit positiver Polung, einer weiteren derartigen Elektrode 31 und einem zwischen beiden Elektroden angeordneten ionenleitfähigen Polymer 32. Die Polymermatrix 33 schließt sich unterhalb der Elektrode 31 an. Das in der Polymermatrix verwendete Polymer weist in seiner Molekülstruktur eine hydrolysierbare Sollbruchstelle auf. In dieser Polymermatrix ist ein Wirkstoff gespeichert.

Durch die Elektrolyse von in der Matrix befindlichen Wassers erhöht sich an der Katode je nach Elektrolysedauer der pH-Wert. Bei einem pH-Wert pH > 7 wird die Wirkstoffmatrix hydrolysiert und der Wirkstoff wird dabei freigesetzt.

Zusätzlich kann die Haut über die textilen Elektrodenstrukturen mit elektrischen Impulsen stimuliert werden, so dass sich Wirkstoffe iontophoretisch übertragen lassen, für die eine solche Stimulation erforderlich bzw. vorteilhaft ist. Die textilen elektrochemisch modifizierbaren Mikroelektrodenstrukturen bieten aber noch weitere Möglichkeiten für einen gezielten Wirkstofftransfer. Durch die elektrochemische Abscheidung von Polymeren in Verbindung mit Wirkstoffen lassen sich die nanoporösen wirkstoffhaltigen Schichten direkt auf den textilen Elektroden abscheiden.

Neben der elektrochemischen Abscheidung von vorgefertigten ionischen Polymeren, können Polymerschichten auf inerten Elektroden auch durch Elektropolymerisation aus Monomeren erzeugt werden. Die durch Elektropolymerisation erzeugten Polymerfilme zeichnen sich durch ein konjugiertes Polymergerüst aus. Dies bildet die Basis für die intrinsische Leitfähigkeit solcher Polymere. Leitfähige Polymerschichten lassen sich chemisch und elektrochemisch oxidieren und reduzieren. Dabei ändert sich die Leitfähigkeit des Polymers um Größenordnungen.

Bei der Änderung des Redoxzustandes ändert sich aber nicht nur die Leitfähigkeit des Polymers. Bei der elektrochemischen Oxidation der Filme werden auf der konjugierten Kette positive Ladungsträger in Form polaronischer und bipolaronischer Zustände erzeugt, deren positive Ladung durch das Einwandern von Anionen oder über das Auswandern von im Film befindlichen Kationen ausgeglichen werden. Ob die Anionen einwandern oder die vorhandenen Kationen den Film verlassen hängt von der Art des Polymers (also von der Wahl des Monomers), von den Bedingungen der elektrochemischen Erzeugung und von der Größe der Ionen ab. So wird bei Polypyrrol das im Beisein von substituierten Toluensulfonaten erzeugt wurde, ein Kationenaustausch beobachtet, wogegen an substituierten Polythiophenen ein Anionenaustausch beobachtet wird.

Diese beiden leitfähigen Polymersysteme stellen sehr stabile Systeme dar, die auch bei häufigen Änderungen des Redoxzustandes keine Alterungserscheinungen zeigen. Insbesondere das von der Firma Bayer entwickelte Poly-3,4-Ethylendioxythiophen (PEDOT) stellt durch die über die Ethylendioxygruppe blockierte Reaktivität der 3- und 4-Position des Thiophenrings ein sehr stabiles leitfähiges bzw. Redoxpolymer dar.

Auf der Basis solcher, galvanisch modifizierter textiler Strukturen bieten sich drei Wege an, ionische Wirkstoffe zu speichern und durch elektrochemische Änderung des Redoxzustandes der leitfähigen Redoxpolymerschicht wieder freizusetzen:
1. Oxidieren einer zum Anionenaustausch neigenden Redoxpolymerschicht im Beisein eines anionischen Wirkstoffes,
2. Elektropolymerisation eines zum Kationenaustausch neigenden Redoxpolymers im Beisein eines kationischen Wirkstoffes und großer Anionen wie substituierten Tolouensulfonaten und
3. Oxidation einer zum Kationenaustausch neigenden Redoxpolymerschicht in Kontakt mit einer Polymerschicht, die mit Wirkstoffkationen beladen ist und die eine stärkere Affinität zu den Kationen aus der Redoxpolymerschicht besitzt.

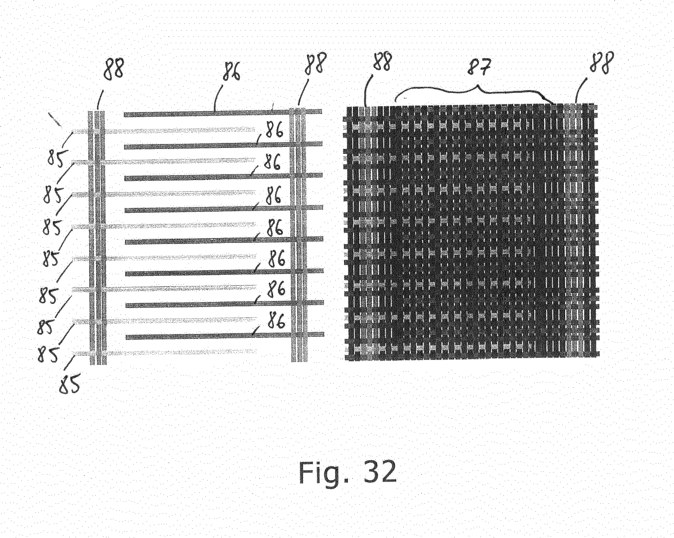

Für die Realisierung dieser schaltbaren Wirkstoffspeicher kommen gewebte Strukturen gemäß Fig. 1 oder auch Fig. 32 oder auch textile Sandwich- bzw. Schichtstrukturen zum Einsatz. Eine der beiden leitfähigen textilen Elektroden ist dazu mit einem Redoxpolymer präpariert. Die Präparation kann im Anschluss auf der gewebten Fläche, wenn es sich um mehrlagige Gewebe handelt, erfolgen. Es ist aber auch möglich, redoxpolymermodifizierte Elektrodengarne zu erzeugen und daraus Gestricke, Gewebe und dergleichen textile Strukturen herzustellen.

Die jeweils verwendete textile Gegenelektrode muss, um das Gesamtsystem schalten zu können, ebenfalls mit einem reversiblen nichtlöslichen Redoxystem ausgerüstet werden. Ohne Probleme kann bereits ein mit Ag/AgCl modifiziertes Ag/Polyamidgarn verwendet werden. Bei der Veranschaulichung der Prozesse in den Schichten in den folgenden Figuren wird deshalb lediglich dieses System verwendet.

Um den Redoxzustand der beiden auf der textilen Struktur befindlichen Redoxschichten (das Redoxpolymer und die Ag/AgCl-Schicht) verändern zu können, müssen diese über ein ionenleitfähiges Material, zweckmäßigerweise einen Elektrolyten, miteinander in Kontakt stehen. Dazu kann der in der Interdigitalstruktur zur Isolation der jeweiligen anderen textilen Kammelektrode eingesetzte Faden bzw. bei einer mehrlagigen Schichtstruktur die zwischen den textilen Elektrodenflächen in den Mehrlagengeweben liegenden Kett- und Schussfäden, mit einem Elektrolyten getränkt, mit einem Elektrolytgel präpariert oder die gesamte Interdigitalstruktur mit einem ionenleitfähigen Polymer beschichtet oder laminiert werden.

Soll der direkte Hautkontakt mit dem auf der Gegenelektrode befindlichen Redoxsystem vermieden werden, können die Redoxsysteme auch auf zwei separate nachgalvanisierte Ag/Polyamid-Textilstrukturen aufgebracht werden, die dann zusammen mit der ionenleitfähigen Folie, die gleichzeitig als Haftvermittler wirkt und bei ca. 130 °C bis 140 °C aufschmilzt, kaschiert werden kann. Fig. 11 veranschaulicht einen solchen Schichtaufbau schematisch. Dieser umfasst eine mit Silber bzw. Silberchlorid präparierte textile Elektrode 34, eine ionenleitfähige Schicht 35 und eine Schicht 36, die mit einem Redoxpolymer präpariert ist. Diese enthält einen anionischen Wirkstoff 37a, der an positive polaronische Abschnitte 37b des Redoxpolymers angelagert ist.

Im Fall der textilen Interdigitalstruktur versinnbildlicht das Schnittprofil zwei benachbarte textile Kammelektroden, die über den mit Elektrolyt präparierten Isolationsfaden oder die auf ihnen auflaminierte ionenleitfähige Folie in ionischem Kontakt stehen.

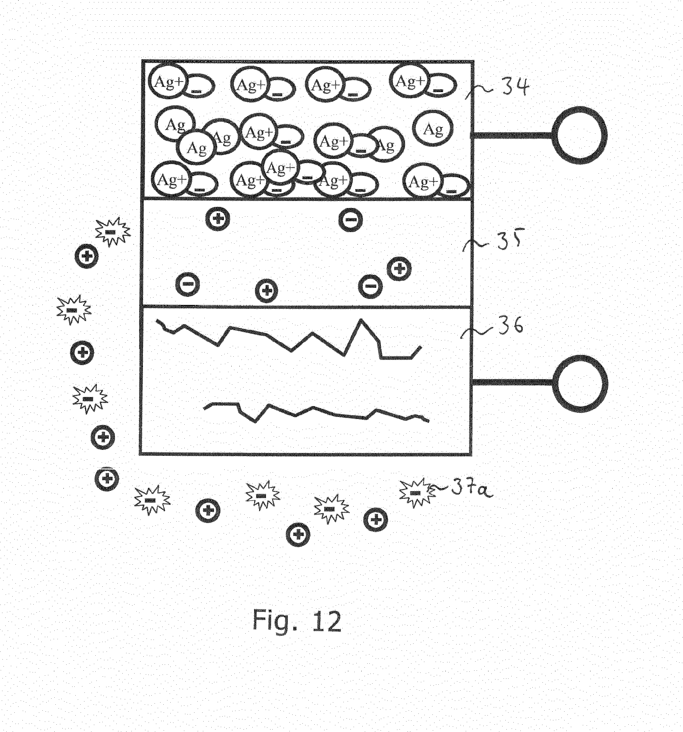

Um einen anionischen Wirkstoff 37a in die Redoxpolymerschicht einzulagern, wird die textile Elektrodenstruktur nach dem elektrochemischen Abscheiden der Redoxpolymerschicht im reduzierten Zustand aus dem organischen Elektrolyten genommen, gewaschen und in den wässrigen anionenhaltigen Wirkstoff gebracht wie in Fig. 12 gezeigt. In diesem Zustand befinden sich die anionischen Wirkstoffionen zusammen mit positiven Gegenionen 38 in der Lösung.

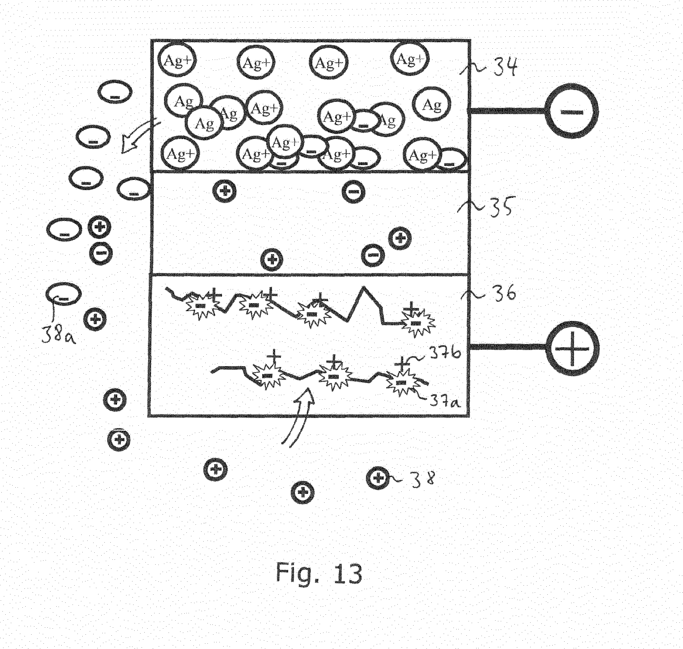

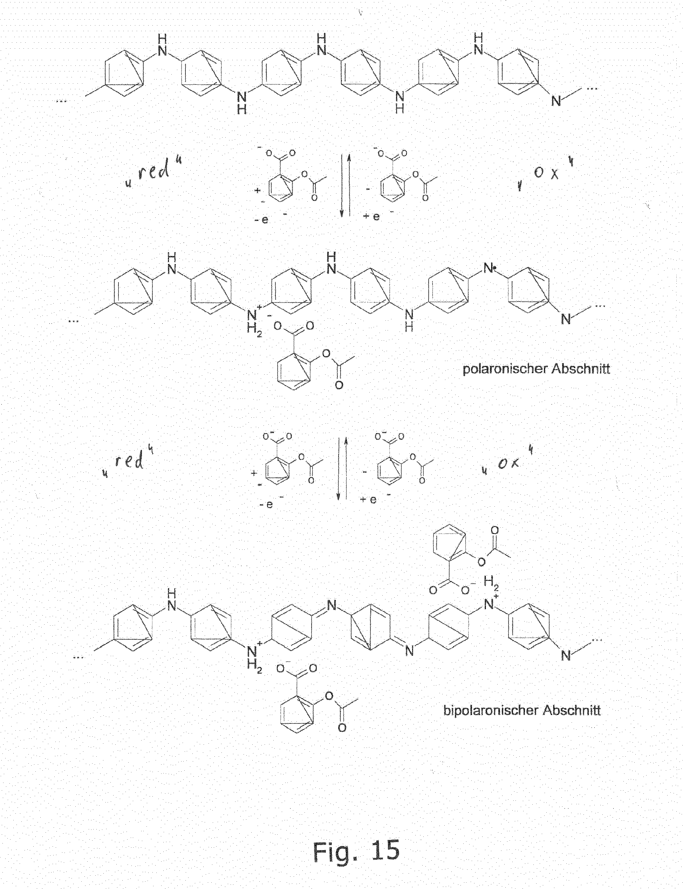

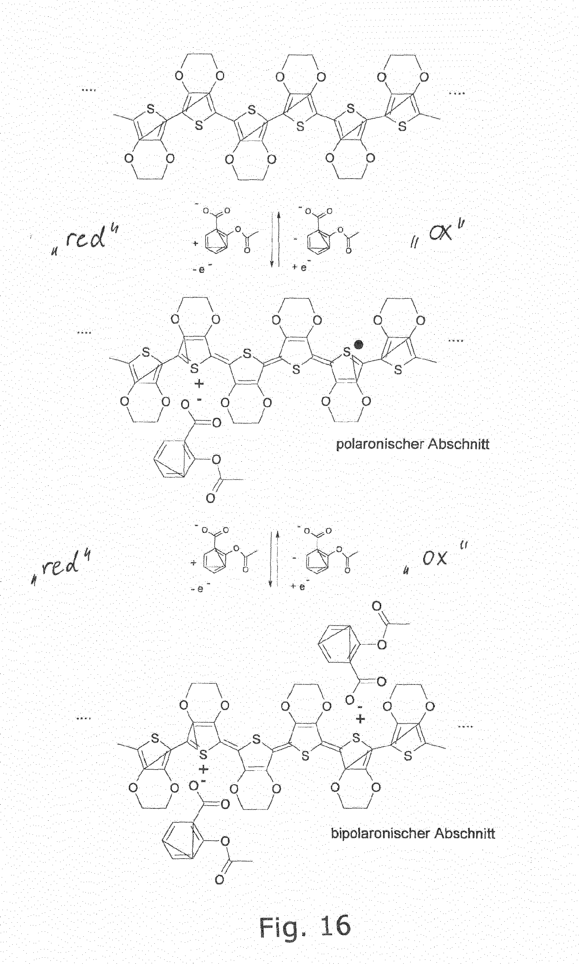

Wird das Redoxpolymer nun als Anode geschaltet, werden im Polymer polaronische und bipolaronische Strukturen auf dem Polymergerüst erzeugt. Beispielhafte molekulare Konfigurationen sind hierzu in den Figuren 15 und 16 dargestellt. Deren positive Ladungen werden nun durch das Einlagern von Anionen, in dem hier gegebenen Fall das Einlagern der anionischen Wirkstoffmoleküle 37a, ausgeglichen. Dies ist in Fig. 13 beispielhaft dargestellt. Die anionischen Wirkstoffmoleküle gehen dabei von der Lösung in die redoxpolymermodifizierte Schicht 36 über. Gleichzeitig wandern Chloridionen 38a aus der Ag/AgCl-Elektrode 34 in die Lösung ab und gleichen dort die Ladung der Gegenionen aus.

Die Bedingungen, unter denen der Wirkstoff optimal und in ausreichender Menge eingelagert werden kann, werden nachfolgend für Acetylsalicyl- und Nicotinsäure beispielhaft erläutert. Zu diesem Zweck werden zunächst für einfache textile Strukturen mit leitfähigen Polymeren geeignete Bedingungen dargestellt, unter denen optimale Redoxpolymerschichten präpariert werden können, die im Kontakt mit dem Wirkstoff und unter Anlegen des für die Wirkstoffeinlagerung notwendigen Potentials stabil bleiben.

Nach dem Beladen der textilen Redoxpolymerschicht wird diese aus der wirkstoffhaltigen Elektrolytlösung entnommen. Im Fall der Verwendung einer textilen Interdigitalstruktur liegt die Grundstruktur des textilen interaktiven transdermalen therapeutischen Systems TI-TTS bereits fertig vor.

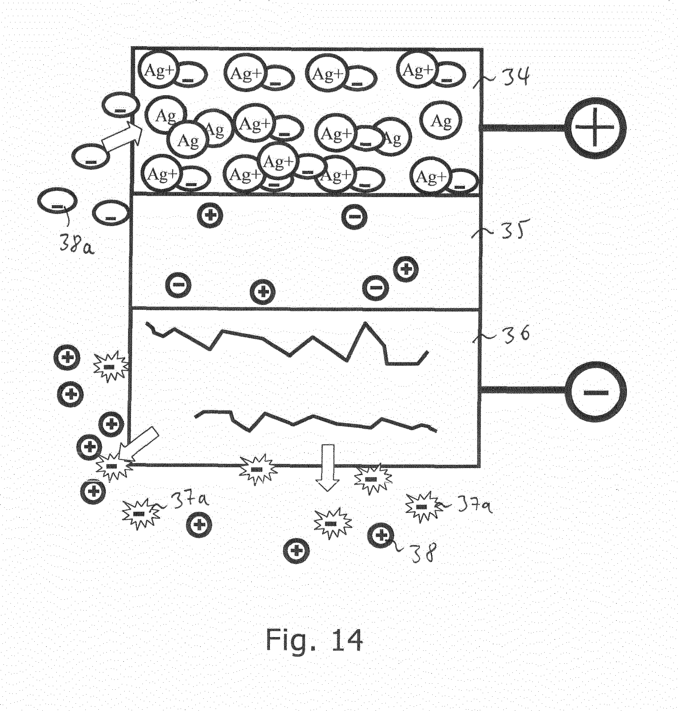

Wird die redoxpolymermodifizierte Elektrode der Struktur als Anode und die Ag/AgCl-Schicht als Katode geschaltet, bleibt der Wirkstoff in der Polymerschicht an die dort erzeugten polaronischen Abschnitte gebunden. Bei umgekehrter Polung, wie in Fig. 14 gezeigt, werden die polaronischen und bipolaronischen Zustände auf der Polymerkette reduziert. Ihre positive Ladung wird ausgeglichen und die Bindung zu den Wirkstoffionen wird aufgehoben. Der anionische Wirkstoff wird freigesetzt. Die Wirkstoffionen 37a treten dann aus der redoxpolymermodifizierten Schicht in die Umgebung, d.h. zum Beispiel auf ein Hautareal, aus. Dabei werden an der anderen Kammelektrode der Interdigitalstruktur nach Fig. 1 Silber zu Silberchlorid oder Silberoxid oxidiert.

In den Figuren 15 und 16 wird die Wirkstoffspeicherung, wie sie in den Fig. 12 bis 14 schematisch beschrieben wird, jeweils am Beispiel der Redoxpolymere Polyanilin (PAni) und Poly(3,4-ethylendioythiophen) (PEDOT) beschrieben. Das bei der Oxidation des Polymers eingelagerte Wirkstoffanion ist in diesem Beispiel Acethylsalicylat. Die Figuren zeigen hierzu die entsprechenden Reaktionsgleichungen und eine den jeweiligen Zustand des Redoxpolymers beschreibende mesomere Grenzstruktur. Die jeweils rechts von oben nach unten aufgeführten Reaktionen stellen dabei die Oxidationen des Redoxpolymers, die links von unten nach oben verlaufenden Reaktionen die Reduktionen des Redoxpolymers dar.

Der Prozess der Speicherung und Wirkstofffreisetzung kann in einem in einer Küvette befindlichen Elektrolyten getestet werden. Die Küvette kann zusätzlich mit einer Gegen- und Referenzelektrode ausgestattet werden, so dass das Elektrodenpotential der mit Wirkstoff beladenen Arbeitselektrode genau eingestellt werden kann. Wird das Potential an der Redoxpolymerschicht erhöht, kann mit spektroskopischen Mitteln verfolgt werden, bei welchem Potential es in der Lösung zum Wirkstoffausstoß kommt.

Zur Untersuchung der Möglichkeiten der Wirkstoffspeicherung können mit Redoxpolymeren modifizierten Garnelektroden auf Fadenelektroden aus galvanisch metallisierten Polyamid-Garnen Redoxpolymerschichten durch Elektropolymerisation erzeugt werden. Derartige Garne sind beispielsweise unter dem eingetragenen Warenzeichen ELITEX verfügbar. Dazu werden versilberte Polyamid-Garne zusätzlich vergoldet bzw. platiniert. Anschließend werden isolierte Proben dieser Elektroden aus Fäden dieser Garne durch ein Einlaminieren in thermischen Laminierfolien angefertigt.

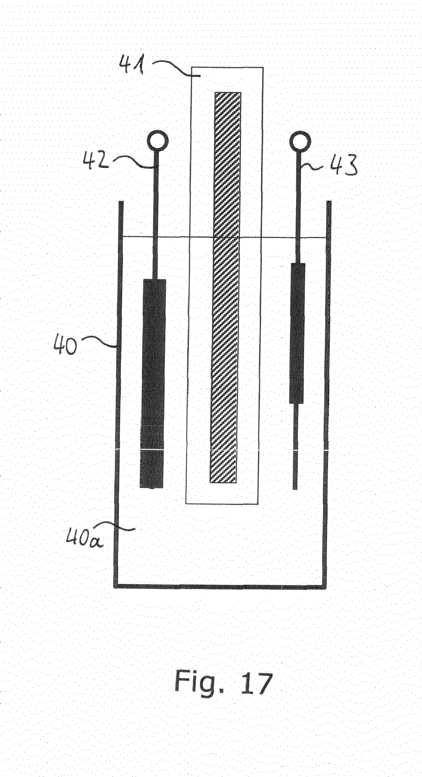

Ein Beispiel hierfür ist in Fig. 17 gezeigt. Die Figur zeigt eine Küvette 40, die mit einer Lösung 40a gefüllt ist. In dieser Lösung befindet sich eine einlaminierte Fadenelektrode 41 mit einer CE-Elektrode 42 sowie einer RE-Elektrode 43. Auf diese Weise können Fadenelektroden mit einer definierten Elektrodenoberfläche untersucht werden.

Zur Fertigung derartiger Versuchselektroden wird das textile Grundmaterial mit Hilfe von durch eine Dreielektrodentechnik kontrollierten Elektrodenpotentialen mittels der Elektropolymerisation mit Redoxpolymerschichten modifiziert. Die Elektropolymerisation erfolgt sowohl unter konstantem Potential, als auch mit Potentialpulsen. Allgemein kommen potentiodynamische Bedingungen zur Anwendung, wie sie für die Erzeugung von Redoxpolymerschichten auf inerten Elektroden für die verschiedensten intrinsisch leitfähigen Polymere bekannt sind.

Anschließend können die mit der Laminiertechnik hergestellten modifizierten Fadenelektroden mit elektrochemischen Methoden wie beispielsweise einer zyklischen Voltammetrie (CV Cyclic Voltammetry) oder einer elektrochemischen Impedanzspektroskopie (EIS Electrochemical Impedanz Spectroscopy) bezüglich ihres Redoxverhaltens charakterisiert werden. Die Laminierung ist zweckmäßig, um zu vermeiden, dass der Elektrolyt der Lösung in der Küvette durch die Kaplillarwirkung in die Multifilamentlösungen eindringt, und die Kontaktklemmen, mit denen die Elektroden mit dem Potentiostaten verbunden werden, benetzt. Dadurch bleibt das Potential an der Fadenoberfläche kontrollierbar und das Messsignal wird nicht zusätzlich durch Redoxprozesse an den Kontaktklemmen überlagert.

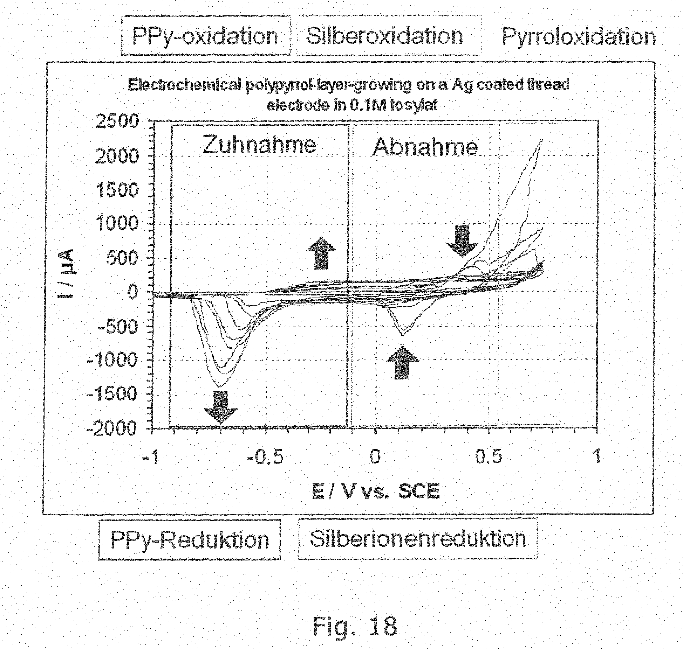

Die an den mit Redoxpolymeren modifizierten inerten Fadenelektroden aufgenommenen Fadenelektroden zeigen die für inerte modifizierte Elektroden typischen, in Fig. 18 beispielhaft angegebenen zyklischen Voltammogramme (CVs). Die Dicke der aufgebrachten Goldschicht sollte zweckmäßigerweise mindestens 100 nm aufweisen. Solche mit elektrochemisch abgeschiedenen Redoxpolymerschichten präparierte Polyamid-Garne können auf einer Anlage zur Versilberung von Polyamidgestricken gefertigt werden. Galvanisch versilberte Rundgestricke können auf der gleichen Anlage definiert galvanisch vergoldet werden. Gold oder Platinbeauflagungen mit Schichten von mehr als 100 nm sind allerdings kostspielig. Für Versuchszwecke empfiehlt sich der Einsatz von ca. 30 cm langen Gestricken, die auf einer diskontinuierlichen Laboranlage galvanisiert und elektropolymerisiert werden können.

Von einer Reihe von Redoxpolymeren wie z.B. PAni, PEDOT und PPy sind wässrige und lösemittelhaltige Dispersionen mit unterschiedlichen Zusammensetzungen, Polymerisationsgraden und Konzentrationen und demzufolge unterschiedlichen elektrischen Leitfähigkeiten verfügbar. Mit Hilfe dieser Dispersionen lassen sich Rundgestricke mit Redoxpolymeren beauflagen. Die Qualität der Präparationsauflagen variiert entsprechend stark und unterscheidet sich in der Haftung, der Leitfähigkeit und dem Redoxverhalten der Filme je nach Zusammensetzung der Dispersion und der zusätzlich verwendeten Binder.

So können beispielsweise unter den eingetragenen Warenzeichen ORMECON oder BAYRON erhältliche Dispersionen verwendet werden. Auf der Basis derartiger Dispersionen können laminierte Faden und Gestrickelektroden präpariert werden, deren Redoxverhalten cyclovoltammetrisch charakterisiert werden kann.

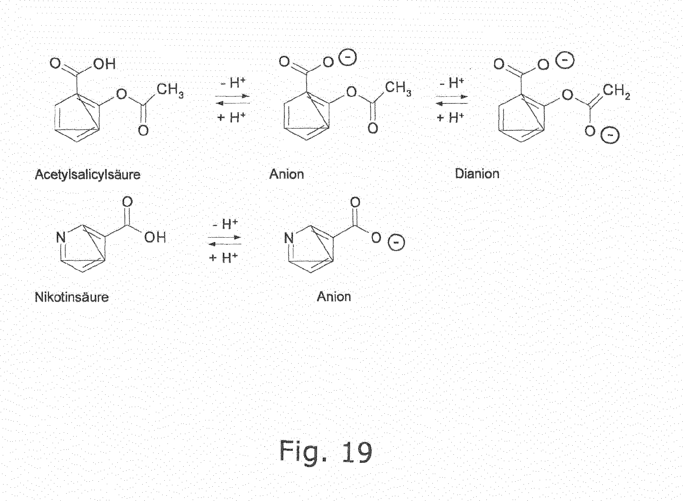

Aus den Geweben lassen sich durch Einlaminieren kleiner Gewebeproben wiederum Testelektroden fertigen, deren Redoxverhalten cyclovoltammetrisch charakterisiert und deren redoxchemisches Aufnahmevermögen für Wirkstoffe spektrochemisch beurteilt werden kann. Dies erfolgt beispielhaft mit dem Modellwirkstoff Acetylsalicylsäure bzw. Nikotinsäure. Fig. 19 stellt hierzu die Dissoziationsgleichungen sowohl für Acetylsalicylsäure als auch für Nikotinsäure dar. Wie aus der Figur zu entnehmen ist, liegt der Säurerest der Acetylsalicylsäure sowohl als einfaches Anion als auch als Dianion vor. Von Nikotinsäure ist nur ein Anion als Säurerest verfügbar.

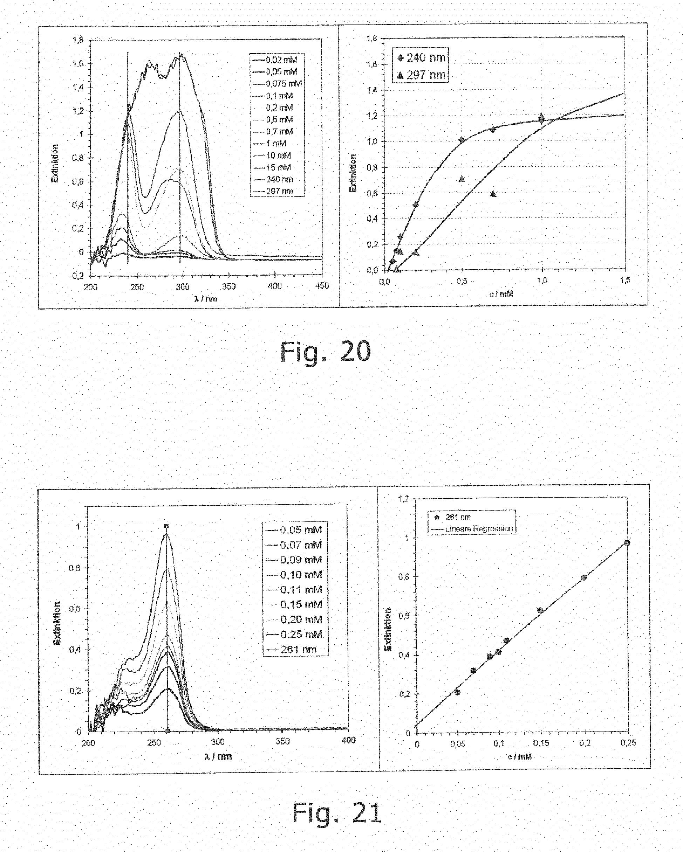

Die hier erwähnten Säurereste zeigen starke Absorptionen im UV-Bereich, wie in Fig. 20 und 21 beispielhaft gezeigt ist. Fig. 20 zeigt hierzu konzentrationsabhängige UV-VIS-Spektren von Acetylsalicylsäure in 0,1 molarer NaCL-Lösung und die zugehörige konzentrationsabhängige Extinktion bei den Wellenlängen 240 nm und 297 nm. Fig. 21 zeigt die konzentrationsabhängigen UV-VIS-Spektren von Nicotinsäure in 0,1 molarer NaCl und die konzentrationsabhängige Extinktion bei 261 nm. Daher kann der Nachweis der Anionen für beide Substanzen in Lösung durch spektroelektrochemische Untersuchungen an den laminierten Faden-, Gestrick- und Gewebeelektroden erbracht werden. Dabei ist zu beachten, dass für quantitative Auswertungen die pH-Wertabhängigkeit der Spektren berücksichtigt werden muss. Die Veränderung der Spektrenform in Abhängigkeit des pH-Wertes resultiert aus der unterschiedlichen Lichtabsorption des Anions und der undissoziierten Wirkstoffsäuren.

Dabei ist insbesondere bei der Acetylsalicylsäure zu berücksichtigen, dass auch die H-Atome an der CH₃-Gruppe eine Acidität aufweisen, wie bereits in Zusammenhang mit Fig. 19 gezeigt worden ist. Auch diese Dissoziation trägt zur Veränderung des UV-VIS-Spektrums bei. Im Fall der Acetylsalicylsäure liegen somit in Lösung letztlich drei Verbindungen - die undissoziierte, die einfach dissoziierte und die zweifach dissoziierte Form - im chemischen Gleichgewicht vor, die sich durch unterschiedliche UV-Spektren auszeichnen.

Für quantitative Auswertungen ist folglich das erhaltene Spektrum in die Anteile der einzelnen Komponenten zu zerlegen oder aber die Kalibrierung in Pufferlösungen bei definierten pH-Werten und folglich konstanter Zusammensetzung auszuführen. Für den Nachweis des Wirkstoffausstoßes aus den polymermodifizierten textilen Elektroden ist dies jedoch zunächst noch nicht erforderlich. Durch den Einsatz einer zusätzlichen Pufferlösung unterscheiden sich die Bedingungen, unter denen die interaktive Wirkstoffabgabe spektroskopisch nachgewiesen wird, zusätzlich von denen, wie sie später auf der Haut zum tragen kommen. Aus diesem Grund ist als Elektrolyt, in dem die elektrochemisch induzierte Wirkstoffabgabe aus den modifizierten textilen Elektroden untersucht wird, eine 0,1 molare NaCl-Lösung zweckmäßig. Sie stellt in erster Näherung die Elektrolytkonzentration im Schweiß auf der Haut nach.

Die im Zusammenhang mit den Figuren 17 und 18 beschriebenen einlaminierten textilen Elektroden aus Polyamid-Fäden und Gestricken lassen sich einfach handhaben und können in einer 1 cm x 1 cm x 4 cm Küvette untergebracht werden, ohne den Strahlengang durch die Küvette zu behindern. Die textilen Elektroden werden zusammen mit einer Referenzelektrode 43, einer Gegenelektrode 42, einem Magnetrührer und einer Inertgaseinleitung so untergebracht, dass die Küvette mit einer 0,1 M NaCl-Lösung aufgefüllt werden kann. Aus den Kalibrierungsspektren aus den Figuren 20 und 21 ist ersichtlich, dass Wirkstoff-Konzentrationen in der Lösung im Bereich von 0,02 bis 0,5 mM UV-VIS-spektroskopisch problemlos detektiert werden können.

Beträgt das Volumen der Küvette beispielsweise 3 ml, dann muss folglich eine Wirkstoffmenge von ca. von 10 µg - 270 µg Acetylsalicylsäure bzw. 7 µg - 185 µg Nicotinsäure aus der laminierten polymermodifizierten textilen Elektrode mit einer Fensterfläche von 0,75 cm² an die Lösung abgegeben werden können, um diese sicher nachzuweisen. Wenn man davon ausgeht, dass die bei der Oxidation der Redoxpolymerschicht auf der Polymerkette erzeugten positiven Ladungen in Form von Polaronen über mindestens vier Monomereinheiten delokalisiert sind, kann maximal ein Wirkstoffanion pro vier Monomereinheiten gebunden werden. Wird als Redoxpolymer Polyanilin (PAni) eingesetzt, muss die textile Elektrode bei einer Fläche von 0,75 cm² mit mindestens 22 µg - 560 µg PAni beauflagt sein. Das entspricht einer Präparationsauflage 0,3 g/m² - 8 g/m². Liegt das Gewebe mit einer Flächemasse von 255 g/m² vor, so sind die Gestrickelektroden mit einer Präparationsauflage von mindestens 0,1 % - 3% zu beauflagen. Geht man davon aus, dass eine vollständige Oxidation der auf die textilen Materialien aufgebrachten Präparationen nicht realistisch erscheint, so zeigt diese Abschätzung bereits, dass auch dann, wenn nur 10 % der Aufnahmekapazität des Redoxpolymers ausgeschöpft werden, eine Präparationsauflage von 1 % bis 30 % ausreicht, um die interaktive Wirkstofffreisetzung spektroskopisch nachweisen zu können.

Die folgende Tabelle fasst einige Abschätzungen der nötigen Präparationsauflage in Form des Redoxpolymers Polyanilin für die Speicherung einer UV-VIS-spektroskopisch nachweisbaren Wirkstoffmenge aus einer textilen 235dtex/f34-Gestrickelektrode aus Polyamid mit einer Fläche von 0,75 cm² zusammen:

| C_{Küvette} mM | n_{Wirkstoff} µmol | m_{Wirkstoff} µg | Kapazität mg/dm² | m_{min_Redoxpolymer} µg | min. Präp-Auflage |
|---|---|---|---|---|---|
| 0,02 | 0,06 | 10,8 | 1,4 | 22,3 | 0,1% |
| 0,5 | 1,5 | 270,3 | 36,0 | 558,6 | 2,9% |

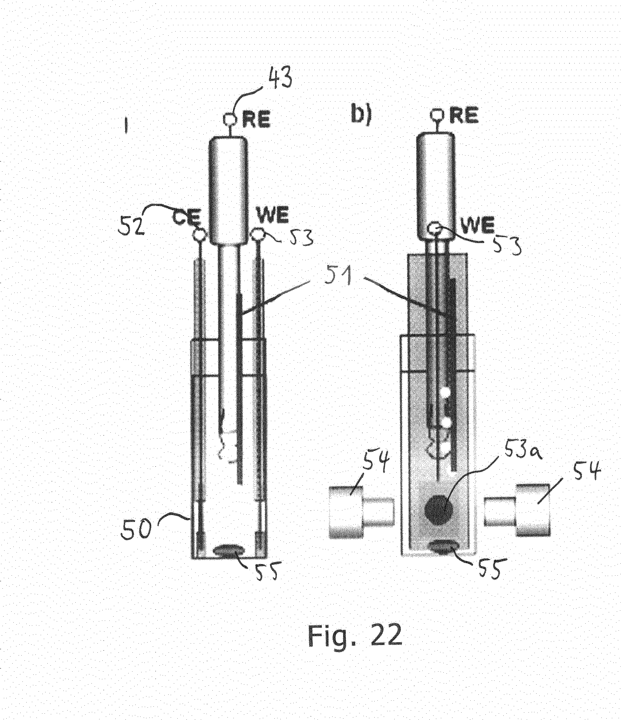

Für den UV-VIS-spektroskopischen Nachweis des elektrochemisch induzierten Wirkstoffausstoßes können folglich einlaminierte laminierte textile Elektroden in einer 1 cm x 1 cm x 4 cm Küvette zum Einsatz gelangen. Zu diesem Zweck ist eine in Fig. 22 beispielhaft dargestellte Messzelle 50 verwendbar, in der die zuvor mit den entsprechenden Redoxpolymeren modifizierten und mit Wirkstoff präparieten textilen Polyamid-Elektroden platziert werden können. Die Messzelle enthält eine Referenzelektrode 43, eine Inertgaseinleitung, insbesondere eine Stickstoffeinleitung 51, eine Gegenelektrode 52, die als Probe vermessene Wirkstoffelektrode 53 mit einem Elektrodenfenster 53a und eine UV/VISspektroskopische Optik 54 zum Realisieren eines durch die Lösung gerichteten Strahlenganges. Ein Magnetrührer 55 gewährleistet eine homogene Durchmischung der Lösung.

Die Präparation mit Redoxpolymeren erfolgt z.B. unter Verwendung kommerziell erhältlicher Dispersionen. Polyanilinschichten können mit Hilfe ausgewählter ORMECON®-Dispersionen sowohl aus wässrigen und lösemittelhaltigen Dispersionen abgeschieden werden. Zur Präparation der textilen Polyamid-Elektroden mit PEDOT ist eine wässrige BAYTRON-Dispersion verwendbar.

Im ersten Schritt werden die Präparationen durch Tauchen auf die bereits laminierten Polyamid-Gestrickelektroden aufgebracht, bei Raumtemperatur getrocknet und das Redoxverhalten der so modifizierten Elektroden cyclovoltammetrisch bei Potentialvorschubgeschwindigkeiten von 20 mV/s - 100 mV/s charakterisiert. Dispersionen, bei denen das für Redoxpolymere typische Verhaltnen zu erkennen ist und das Signal über mehrere Zyklen erhalten bleibt, sind für den spektroelektrochemischen Nachweis der elektrochemisch induzierten Wirkstoffabgabe besonders geeignet.

Die Präperationsauflagen können durch Differenzwägung vor der Präparation und nach dem Trocknen bestimmt werden. Die Beauflagungen liegen zwischen 1 % und 30 %. Hohe Beauflagungen, etwa durch mehrfache Präparation, zeigen zwar größere Stromsignale, die aber die charakteristische Form der für die eingesetzten Redoxpolymere typischen Cyclovoltammogramme nicht mehr zeigen und deren Signalhöhe häufig nach wenigen Redoxzyklen deutlich abnimmt. Aus diesem Grund und auch als Resultat einer Abschätzung, dass bereits eine Präparationsauflage von 0,1 % bis 3 % bei der eingesetzten Elektrodengröße ausreicht, um den Wirkstoffausstoß nachzuweisen, kommen zunächst nur einfach präparierte Elektroden zum Einsatz.

Die präparierten textilen Elektroden werden zunächst in der elektrochemischen Messzelle 50 elektrochemisch in einer 0,1 M NaCl-Lösung in Gegenwart der Stickstoffeinleitung 51 reduziert, wobei mit Hilfe der Dreielektrodentechnik das Potential der Elektrode über eine Referenzelektrode 52 (gesättigte Kalomelelektrode (SCE)) kontrolliert wird. Nach einem deutlichen Abfall des Stromes und einer deutlichen Entfärbung der Redoxpolymerschicht werden die Elektroden bei anliegendem Potential entnommen und direkt in die bereitete Wirkstofflösung gegeben. Dort werden während der Präparation mehrere Elektroden aufbewahrt. Bereits nach dem Eintauchen der Elektroden in die Wirkstofflösungen färben sich die Redoxpolymerfilme auf den textilen Elektroden je nach eingesetztem Polymer wieder dunkelgrün (PAni) oder blau (PEDOT). Dies deutet auf die Oxidation der Filme durch den in der Wirkstofflösung gelösten Sauerstoff hin.

Bei dieser chemischen Oxidation der Redoxpolymere werden Polaronen bzw. Bipolaronen auf der Polymerkette erzeugt, deren positive Ladung durch die sich in den Film einlagernden Wirkstoffionen ausgeglichen werden. Anschließend werden die Redoxpolymerfilme in der elektrochemischen Zelle oxidiert, wobei die Wirkstofflösung als Elektrolyt wirkt. Auf diese Weise werden zusätzliche positive Ladungen auf der Polymerkette erzeugt, die weitere Wirkstoffanionen binden.

Die so erzeugten polymer- und wirkstoffmodifizierten textilen Elektroden werden abschließend mit deionisiertem Wasser gewaschen und in die spektroelektrochemische Zelle zum Nachweis der interaktiven Wirkstoffabgabe eingesetzt. Bereits beim Einführen der Elektroden kann ein Anstieg der Wirkstoffkonzentration nachgewiesen werden. Beim Anlegen des negativen Potentials, bei dem der Polymerfilm reduziert wird, und die Wirkstoffionen folglich nicht mehr chemisch an die nicht mehr existierende polaronische bzw. bipolaronische Struktur der Polymergerüstes gebunden sind, kommt es wie erwartet zu einer UV-VIS-spektroskopisch nachweisbaren Erhöhung der Wirkstoffkonzentration in der Küvette um ca. 10 % bis 20 %. Damit kann nachgewiesen werden, dass sich Wirkstoffionen in Redoxpolymer bei der elektrochemischen Oxydation als Gegenionen einlagern lassen.

Um die chemischen Einlagerung von Wirkstoffionen in Redoxpolymere und deren interaktive elektrochemische Freisetzung für textile TTS nutzen zu können, müssen folgende Voraussetzungen erfüllt sein:
- eine möglichst gute Haftung der Redoxpolymerfilme auf den Garnelektroden,
- eine Vergrößerung der Präparationsauflage ohne Verlust des Redoxverhaltens,
- ein effektives Beladen der Redoxpolymerschichten mit dem vorgesehenen Wirkstoff,
- ein effektives Reinigen der präparierten Elektroden von nicht chemisch gebundenem Wirkstoff.

Um eine bessere Haftung der Redoxpolymerauflagen zu erzielen, können die präparierten Elektroden thermisch behandelt werden. Möglich ist auch eine Thermosoierung und Präparation der Polyamid-Gestricke mit Redoxpolymerdispersionen am Foulard analog zur Belegung derartiger Gestricke mit Farbstoffen. Die thermisch behandelten Redoxpolymerauflagen zeigen eine deutlich verbesserte Haftung. Selbst an mehrfach auf diese Weise präparierten Gestrickelektroden oder solchen aus präparierten Gestricken gefertigten Elektroden ist das für Redoxpolymere typische cyclovoltammetrische Verhalten noch nachweisbar.

Dispersionen, die zunächst beim Trocknen bei Raumtemperatur schlechte Ergebnisse lieferten, zeigen unter Umständen deutlich bessere Resultate, als die zuvor besseren Produkte, so dass diese Dispersionen bei der Optimierung wieder zu berücksichtigen waren.

Um die Redoxpolymerschichten effektiver mit Wirkstoffen zu beladen, können die Konzentrationen und der pH-Wert der Lösungen variiert werden. Einen entscheidenden Effekt übt dabei die Waschlösung auf die modifizierten Elektroden aus.

So wird nach dem Eintauchen der wirkstoffbeladenen Elektroden nach dem Waschen in einer 1 N NaCl-Lösung keine Wirkstoffabgabe sowohl bei angelegtem positiven Potential als auch im potentialfreien Zustand beobachtet, so das der eigentliche Wirkstoffausstoß bei negativem Potential deutlich besser beobachtet werden kann. Wird dagegen die wirkstoffbeladene Elektrode noch zusätzlich unter fließendem Wasser aus der Leitung gespült, wird auch nach angelegtem Potential kein Wirkstoff abgegeben. Untersuchungen haben gezeigt, dass die elektrochemisch durch den Potentialpuls freigesetzte Wirkstoffmenge nach dem Spülen selbst mit 1 M NaCl-Lösung deutlich höher ist als beim Einsatz von deionisiertem Wasser und dass beim Einsatz von Leitungswasser keine Freisetzung auftritt. Natriumchlorid mit einer Konzentration von 0,1 M, wie es im Schweiß der Haut vorliegt, führt folglich nicht zu einer Wirkstoffabgabe.

Während die Versuche mit Natriumchloridlösungen sich als reproduzierbar und nahezu unabhängig von der Konzentration erweisen, sind insbesondere die Versuche mit direkt aus der Anlage zur Deionisation entnommenem Wasser und Leitungswasser wenig reproduzierbar und führen häufig zum Ausbleiben der Wirkstoffabgabe nach dem Anlegen des negativen Potentials. Beim Spülen der wirkstoffbeladenen Elektroden unter fließendem Leitungswasser entfärben sich die Präparationen deutlich. Spült man den Film dagegen mit aus Leitungswasser angesetzten Natriumchloridlösungen, tritt der Effekt nicht auf. Verringert man die Natriumchloridkonzentration, wird selbst bei geringen Konzentrationen keine Entfärbung beobachtet. Füllt man Leitungswasser in ein Becherglas und spült darin die wirkstoffbeladenen polymermodifizierten Elektroden, kommt es ebenfalls zu keiner farblichen Veränderung der Elektroden. Der Effekt wird lediglich direkt an dem aus der Leitung fließenden Wasser mit deutlich geringerem Sauerstoffgehalt oder mit Innertgas gespültem Wasser beobachtet. Die Redoxpolymerfilme werden chemisch reduziert und setzen den Wirkstoff wieder frei. Ist dagegen das Wasser mit Luftsauerstoff gesättigt, kann das wirkstoffhaltige Redoxpolymer nicht chemisch reduziert werden und der Wirkstoff bleibt selbst nach der Aufbewahrung über Nacht bzw. über mehrere Tage im Redoxpolymer ionisch gebunden.

Auf Grund dieser Untersuchungsergebnisse können Polyamid-Gestricke mit guter Haftung mit Polyanilin (PAni) und Poly(3,4-ethylenedioxythiophen) (PEDOT) präpariert und von lediglich an der textilen Struktur anhaftendem nicht chemisch gebundenen Wirkstoff gereinigt werden. Mit der Optimierung der Polymerfilmpräparation und vor allem durch das effektive Auswaschen des nicht chemisch gebundenen Wirkstoffes ohne Reduktion des Polymerfilms erweist sich ein krisförmiges Fenster in der Laminierfolie mit einem Durchmesser von 4 mm (A = 0,126 cm²) aus zweckmäßig.

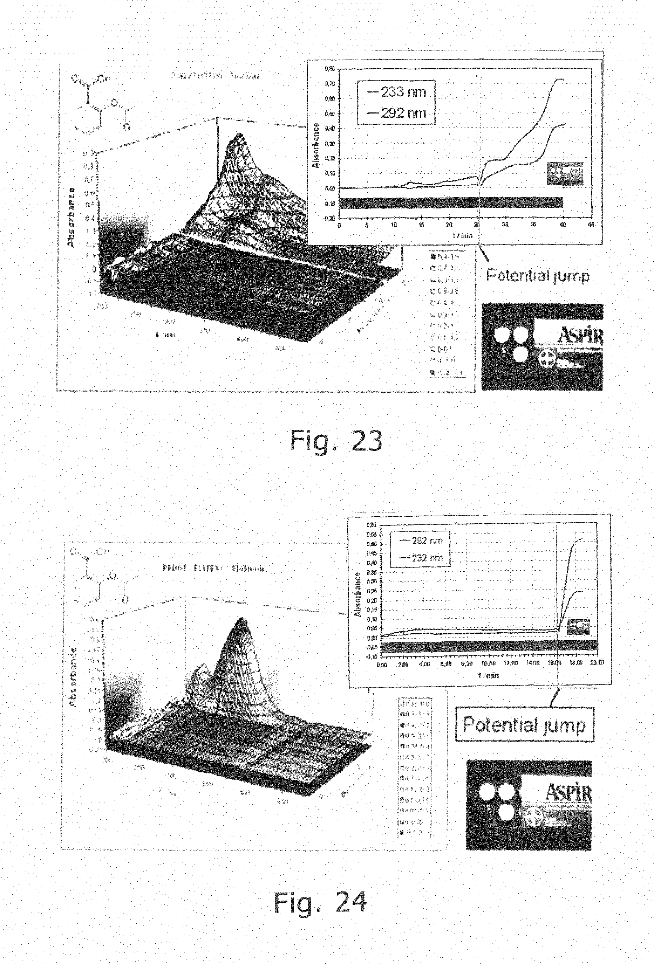

Untersuchungen zur Wirkstoffabgabe sollen anhand der Modellwirkstoffe Acetylsalicylsäure bzw. die Nicotinsäure erläutert werden. Eine typische UV-VISspektroskopische Messung an einer mit PAni modifizierten und mit Acetylsalicylsäure beladenen laminierten textilen Polyamid-Gestrickelektrode zeigen Fig. 23 und 24.

Die während der Freisetzung nach dem Potentialsprung auf -400 mV (vs. SCE) registrierten Spektren von Acetylsalicylsäure verändern deutlich ihre Form, was auf den bei der Wirkstofffreisetzung in der Küvette sich deutlich verändernden pH-Wert zurückzuführen ist. Ursache hierfür ist nicht allein die Freisetzung des Wirkstoffanions, das nach der Freisetzung undissozierte Acetylsalicylsäure bildet, sondern liegt vor allem in den beim Redoxprozess des Polyanilins eingeschlossenen Protonierungsreaktionen begründet. Eine solche Veränderung der Spektren wird aus diesem Grund bei der elektrochemisch induzierten Freisetzung von Acetylsalicylsäure aus PEDOT nicht beobachtet, aufgrund des sich einstellenden chemischen Gleichgewichtes zwischen freier Acetylsalicylsäure, ihrem Anion und dem in Fig. 19 gezeigten Dianion. Aus dem Extinktionsmaximum vom 0,7 kann aber in erster Näherung auf eine Konzentration von mindestens 0,65 mM in der 3 mL Küvette geschlossen werden, was einer freigesetzten Menge von 356 µg Acetylsalicylsäure aus einer textilen Fläche von nur 0,126 cm² entspricht. Aus einem 10 cm x 10 cm großen textilen Pflaster könnten folglich 283 mg Acetylsalicylsäure freigesetzt werden. Dies entspricht in etwa der in einer Kopfschmerztablette enthaltenen Wirkstoffmenge.

Noch exakter lässt sich die Wirkstoffkapazität der polymermodifizierten textilen Polyamid-Elektroden für die mit PEDOT präparierten Gestricke bestimmen. Hier treten während des Freisetzens keine Veränderungen des Spektrums auf, so dass eine Kalibrierung über eine bekannte Acetylsalicylsäurelösung erfolgen kann, bei der die beiden Absorptionspeaks bei 232 nm und 292 nm durch Zugabe von Natriumhydroxidlösung auf das gleiche Verhältnis eingestellt werden. Damit ist für den Modellwirkstoff Acetylsalicylsäure nachweisbar, dass sich dieser als anionisches Gegenion in einem Redoxpolymer, das als Präparationsauflage an galvanisch versilberte Textilien aufgebracht ist, dauerhaft speichern und durch elektrochemische Reduktion des Filmes oder ein geeignetes Reduktionsmittel wieder freisetzen lässt.

Das Schmerzmittel Acetylsalicylsäure ist jedoch für textile interaktive transdermale therapeutische Systeme etwas weniger von Interesse, da für die Applikation von Schmerzmitteln zwar eine kontrollierte, aber nicht unbedingt interaktive Applikation erforderlich ist. Solche Systeme können ohne Probleme erst dann angebracht werden, wenn sie benötigt werden.

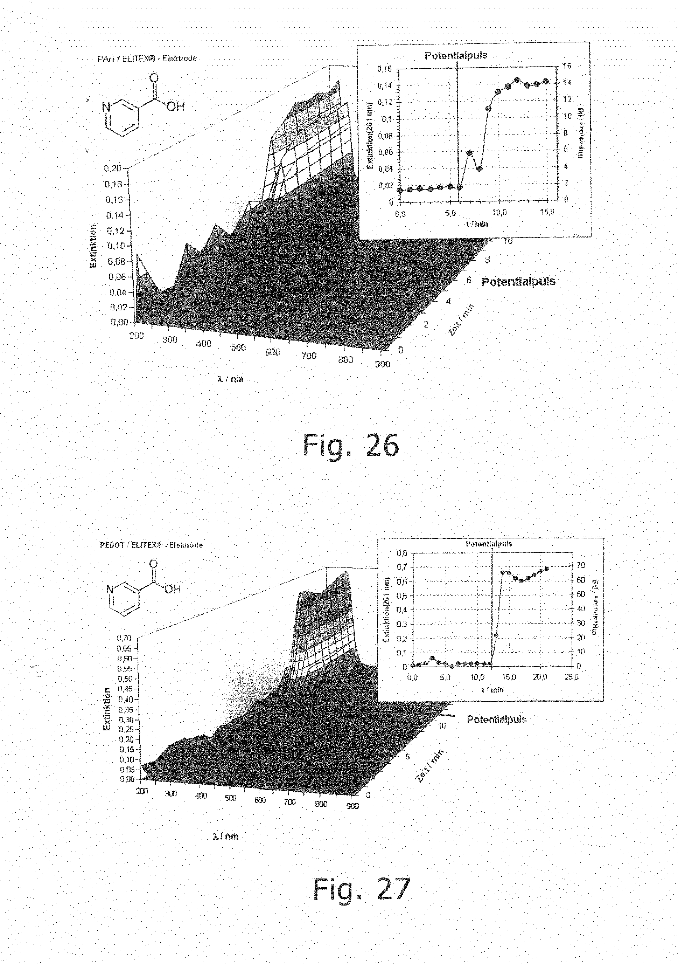

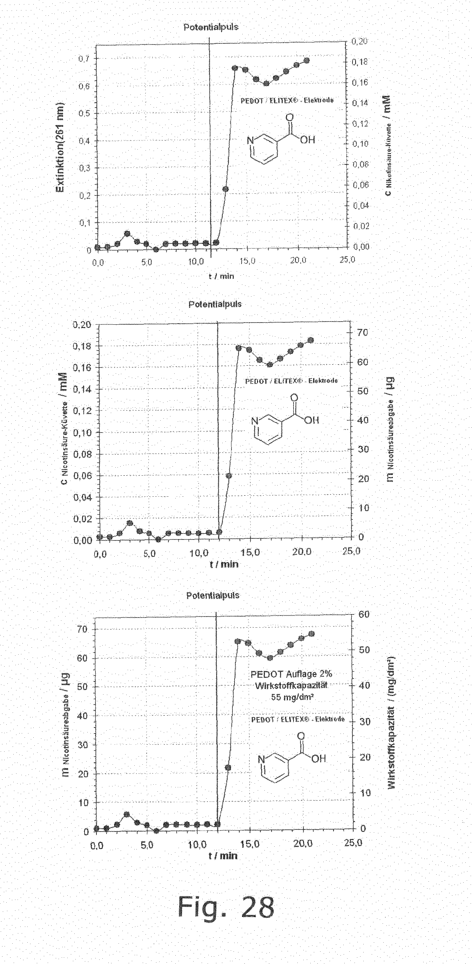

Hingegen ist eine interaktive Applikation von hyperämisierenden Mitteln, also Stimulanzien der Thermorezeptoren der Haut wie z.B. Nicotinsäure von großer Bedeutung, so dass nachfolgend eine interaktive Abgabe von Nicotinsäure beispielhaft dargestellt werden soll. Wie die *in situ* spektroelektrochemischen Untersuchungen an mit wirkstoffbeladenen Redoxpolymeren auf textilen Polyamid-Materialien im Fall des PAni's gemäß Fig. 26 und des PEDOT's gemäß Fig. 27 zeigen, kann eine interaktive Abgabe von Nicotinsäure in ausreichender Menge nach einer entsprechende Optimierung der präparierten Polymerschichten und des Redoxprozesses zur Inkorporierung der Wirkstoffionen in die auf den leitfähigen Textilien aufgebrachten Redoxpolymermatrix realisiert werden. Da die Optimierung der Abscheidungsbedingungen und des elektrochemischen Beladens der beiden Polymere relativ problemlos an die Nikotinsäure angepasst werden kann und auch hier beachtliche Beladungsmengen von 55 mg/dm² gemäß Fig. 28 auf lediglich einfach präparierten Polyamid-Gestricken erreichbar ist, kann dass das Verfahren auch auf weitere Wirkstoffe, die als Säure oder bereits als Anion vorliegen, übertragen werden.

Für textile TTS ist es notwendig, mit Redoxpolymeren präparierte Polyamid-Garne, beziehungsweise bereits mit Wirkstoffen beladene redoxpolymermodifizierte Garne, zu erzeugen.

Die cyclovoltammetrischen Untersuchungen von elektrochemisch polymerisierten Redoxpolymerfilmen und solchen, die aus Dispersionen auf kleinflächigen Polyamid-Garnen und Gestrickelektroden abgeschieden worden sind, zeigen, dass die elektrochemische Polymerisation vor allem dann qualitativ gute Polymerschichten liefert, wenn das Polyamidmaterial mit dünnen (ca. 100 nm) chemisch inerten Metallschichten aus Gold oder Platin überzogen ist.

Bei der direkten elekrochemischen Polymerisation auf die Silberoberfläche der Garnmaterialien muss beim Aufwachsen der Polymerfilme das Potentialregime für die Polymerisation exakt kontrolliert werden. Dabei ist insbesondere ein Potentialpuls zur Initialisierung der Elektropolymerisation nötig. Häufig, insbesondere bei der Abscheidung von Polypyrrol, muss dieser mehrfach wiederholt werden, bis bei niedrigeren Potentialen nach der Erzeugung der ersten Polymerkeime die Elektropolymerisation fortschreitet, ohne dass die Silberschicht angegriffen wird. Für Testelektroden in elektrochemischen Zellen, die mit einem Potentostaten mit Dreielektrodentechnik angesteuert werden, ist es möglich, schonende Abscheidungsmechanismen zu finden, ohne dass die Silberoberflächen merklich angegriffen werden.

Die kommerziell erhältlichen Reoxpolymerdispersionen, die für Polyanilin unter dem Markennamen ORMECON und für Poly(3,4-ethylenedioxythiophene) unter der Marke BAYTRON P vertrieben werden, zeigen eine ausreichende Haftung und ein für Redoxpolymerfilme typisches Redoxverhalten, ohne das beim Oxidieren des Polymerfilms die versilberten Polyamidfäden korrodieren. Es gelingt damit, Wirkstoffe wie Acetylsalicyl- und Nikotinsäure als Gegenionen zu den positiv geladenen Abschnitten auf der Polymerkette zu inkorporieren.

Im Folgenden soll eine beispielhafte Vorgehensweise zur industriellen Herstellung von mit Redoxpolymeren modifizierten Polyamid-Textilien erläutert werden. Zu diesem Zweck werden Polyamid-Rundgestricke mit den erwähnten Redoxpolmerdispersionen an einem Foulard ausgerüstet und anschließend thermisch fixiert. Proben aus den so behandelten Rundgestricken können wiederum in der in beschiebenen Art und Weise in laminierte Testelektroden eingebracht, cyclovoltammtrisch bezüglich ihres Redoxverhaltens untersucht, mit Wirkstoffen beladen und deren Wirkstoffabgabe qualitativ nachgewiesen und mit Hilfe von für unterschiedlich pH-Werte für die jeweiligen Wirkstoffe erstellte Kalibrationskurven auch quantitativ ausgewertet werden.

Um jedoch textile interaktive TTS aufbauen zu können, reicht es nicht aus lediglich die mit Wirkstoffen beladenen textilen Flächen herzustellen. Die in die polymermodifizierten Polyamid - Materialien inkorporierten Wirkstoffe lassen sich nur dann freisetzen, wenn sich textile Strukturen aufbauen lassen, in denen die wirkstoffbeladenen textilen Flächen von einer als Gegenelektrode fungierenden zweiten textilen Fläche separiert sind, aber ionisch mit dieser in Kontakt stehen.

Zweckmäßigerweise sollten die dabei zu realisierenden textilen Wirkstoffpflaster keine zu hohe Konzentrationen eines Elektrolyten bzw. Gel-Elektrolyten aufweisen, sondern nach Möglichkeit bereits durch die beim Tragen auf der Haut vorhandene Restfeuchte in der textilen Struktur funktionsfähig sein. Daher müssen die wirkstoffbeladene Elektrode und die Gegenelektrode in der Struktur möglichst dicht beieinander liegen.

Dies kann man bei einer ersten Ausführungsform dadurch erreichen, indem die textile wirkstoffbeladene Polyamid - Fläche auf eine textile Fläche mit geringer Flächenmasse und hohem Wasseraufnahmevermögen aufkaschiert und diesen Verbund mit einer weiteren Polyamid - Fläche verbunden wird.

Noch effektiver sind aber textile Strukturen in einer Ausführungsform, bei denen die wirkstoffbeladene Elektrode und die Gegenelektrode als verwebte Fäden und Filamente zu textilen Interdigitalstrukturen oder aber mehrlagigen Gewebestrukturen webtechnisch miteinander verbunden sind. Dazu ist es notwendig, dass das mit dem Redoxpolymer modifizierte Polyamid-Material oder aber sogar das mit Wirkstoff beladene Material als verwebbarer Faden ausgebildet ist.

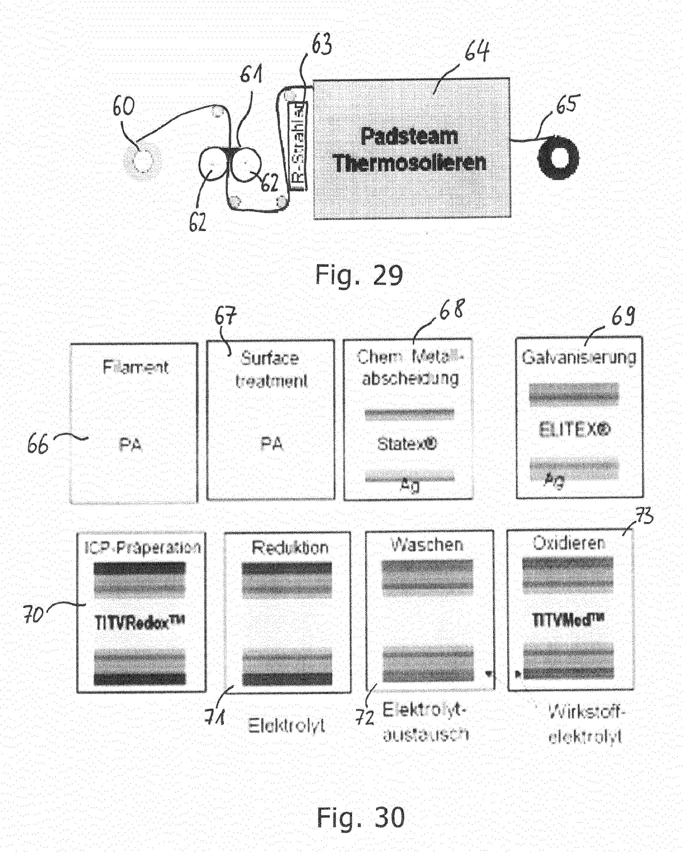

Im Folgenden soll gezeigt werden, wie die gewonnenen Garne zu textilen interaktiven TTS verarbeitet werden können. Hierzu dient eine in Fig. 29 beispielhaft dargestellte Vorrichtung. Das Ausgangsgarn wird von einem Garnvorrat 60, im wesentlichen einer Garnspule, durch eine Polymerdispersion 61 geführt. Diese ist zweckmäßig mit eigenen Fördereinrichtungen, insbesondere Förderrollen 62, versehen. Eine Trocknung erfolgt mit einem IR-Strahler 63, bevor die Polymerbeschichtung in einer Thermosolieranlage 64 fixiert wird. Im Ergebnis wird ein mit Redoxpolymer beschichtetes Garn 65 erzeugt und schließlich aufgespult. Grundsätzlich kann dabei auf eine Vorrichtung zum Färben von Textilien zurückgegriffen werden.

In den folgenden Abschnitten wird beispielhaft gezeigt, wie bereits wirkstoffbeladene Garne in analoger Weise auf einer industriellen Anlage gefertigt werden können und auf welche Weise an den daraus gebildeten textilen Strukturen eine interaktive Wirkstoffabgabe nachweisbar ist.

Ein schematisches Beispiel hierzu ist in Fig. 30 dargestellt. Ein Garnfilament 66 dient als Ausgangsmaterial. Nach einer Oberflächenbehandlung 67 und einer chemischen Metallabscheidung 68, die insbesondere ein Versilbern oder Platinieren ist, erfolgt optional eine Galvanisierung 69, bei der die Metallschicht galvanisch verdickt werden kann. Chemische und galvanische Metallabscheidung können auch alternativ zueinander ausgeführt werden. Das Resultat dieser Behandlungen liegt als metallisierter Faden bzw. als metallisiertes Filament vor.

In einem Schritt 70 erfolgt nun ein Aufbringen des Redoxpolymers aus der Polymerdispersion unter Anwendung einer elektrochemischen Polymerisation. In einem Elektrolytbad wird das Redoxpolymer des Filamentes in einem Schritt 71 reduziert und in einem Schritt 72 gewaschen. Dabei findet ein Elektrolytaustausch statt. Abschließend erfolgt ein elektrochemisches Oxidieren 73 des mit dem Redoxpolymer belegten Filamentes in einer Wirkstofflösung. Der Wirkstoff wird dadurch in das Gerüst des Redoxpolymers aufgenommen und gespeichert. Im Ergebnis liegt nun ein wirkstoffbeladenes redoxpolymermodifiziertes Filament vor, das in textile Strukturen integriert, insbesondere eingewebt, werden kann.

Alternative, jedoch prinzipiell gleichwertige Prozessschritte zur Realisierung des Schichtaufbaus an den Filamenten des Garns können darin bestehen, dass zuerst eine Fertigung eines Rundgestrickes aus einem PA-Garn erfolgt. Dem schließt sich eine chemische Versilberung des Rundgestrickes und/oder eine galvanische Versilberung an. Als nächstes erfolgt ein Foulardieren und Thermosolieren des Rundgestrickes mit Redoxpolymerdispersionen. Es erfolgt danach eine elektrochemische und/oder chemische Reduktion der Redoxpolymerpreparation. Die Rundgestricke werden nachfolgend in einer wirkstoffhaltiger Waschlösung gewaschen. Abschließend erfolgt die elektrochemische und/oder chemische Oxidation der Redoxpolymerpräparation in der wirkstoffhaltigen Lösung. Aus dem so behandelten Rundgestrick können wirkstoffhaltige Garne, aus denen der Wirkstoff interaktiv durch elektrochemische und oder chemische Reduktion freigesetzt werden kann, hergestellt werden.

Die industrielle Fertigung von mit verschiedensten Wirkstoffen beladenen Garnen kann damit über bereits bekannte Prozesse wie sie zur Herstellung der Polyamid - Garne und zum Färben von Textilien zum Einsatz kommen, realisiert werden.

Aus Laborversuchen zur Beladung solcher redoxpolymermodifizierter Textilien mit den Wirkstoffen Acetylsalicyl- und Nikotinsäure können Schlussfolgerungen für die technisch Umsetzung zur Fertigung von wirkstoffbeladenen Garnen, deren Behandlung und Lagerung gezogen werden.

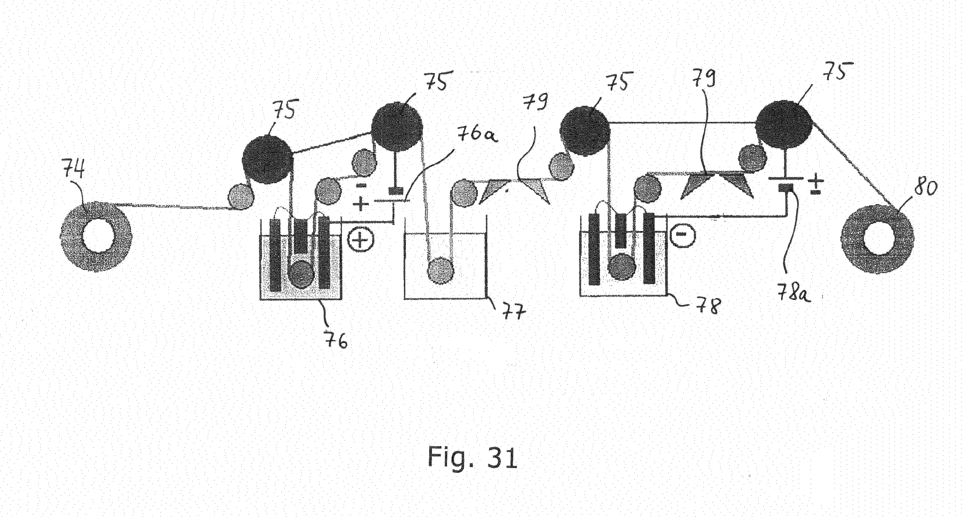

Ein Beispiel für eine Anlage zur kontinuierlichen Herstellung von Rundgestricken bzw. Garnen mit inkroporierten Wirkstoffen, die auf den Erfahrungen bei der Galvanisierung von Rundgestricken zur Produktion von Polyamid -Textilien beruhen, ist in Fig. 31 dargestellt. Ein mit dem Redoxpolymer präparierter Garnvorrat 74, der entweder in Form eines Gestrickes oder als Einzelfaden vorliegt, wird über Fördereinrichtungen 75 durch ein erstes Elektrolytbad 76 hindurchgeführt und von einer Spannungsquelle 76a mit elektrischer Spannung beaufschlagt, wobei eine elektrochemische Reduktion des Redoxpolymers erzeugt wird, wobei Fremdionen aus der Polymermatrix verdrängt werden. Anschließend tritt das Garn wieder aus dem Elektrolytbad heraus, wobei das Redoxpolymer in Kontakt mit dem Luftsauerstoff tritt und dadurch oxidiert. Nun wird das Garn durch ein Wirkstoffbad 77 geleitet, das eine wässrige Wirkstofflösung enthält. Die im oxidierten Zustand befindliche Redoxpolymerschicht nimmt dabei den Wirkstoff im Polymergerüst auf. In einer Vakuumanlage 79 erfolgt ein Trocknen und Entfernen nicht absorbierter Wirkstoffreste von der Oberfläche des Garns. Anschließend kann das Garn ein zweites Wirkstoffbad 78 mit einer weiteren Spannungsquelle 78a durchlaufen, bei dem der Wirkstoffgehalt im Redoxpolymer neu eingestellt werden kann. In einer weiteren Vakuumanlage erfolgt ein abschließendes Trocknen des Garns und ein Sammeln in einem Garnvorrat 80 mit wirkstoffbeladenem redoxpolymermodifiziertem Garn.

Das so modifizierte Garn kann im wesentlichen auf zwei Arten in ein Textil integriert werden. Neben der bereits erwähnten Möglichkeit des Aufbaus eines textilen interaktiven TTS's durch das Kaschieren von einer mit Wirkstoff beladenen textilen Fläche mit einer textilen Fläche mit hoher Feuchtigkeitsaufnahe und/oder ionischer Leitfähigkeit sowie einer textilen Polyamid-Fläche, bietet eine Interdigitalstruktur aus vergleichbaren Garnmaterialien eine Reihe von Vorteilen.

Fig. 32 zeigt unter eine schematische Darstellung einer gewebten Interdigitalstruktur mit einem Elektrodenkamm aus einem wirkstoffbeladenen redoxpolymermodifizierten Polyamid-Garn 85 und einem zweiten Elektrodenkamm aus hochleitfähigem versilberten Polyamidgarn 86. Die dazwischen angeordneten elektrisch nicht leitfähigen Kett- und Schussfäden sind bei dem linken Teilbild zur Veranschaulichung der Grundstruktur weggelassen worden. Das rechte Teilbild zeigt die vollständige gewebte Interdigitalstruktur für ein textiles interaktives transdermales System mit den beiden Elektrodenkämmen. Eine Gesamtheit elektrisch nicht leitfähiger Kett- und Schussfäden 87 bilden eine abstandshaltende Gewebestruktur zwischen den Elektrodenkämmen aus. Eine Gesamtheit aus metallisierten Fäden 88 dient zur Kontaktierung der Kammelektroden.

Die für die Funktion des Textils relevanten Abstände zwischen den einzelnen Fäden der Kammelektroden werden durch die Feinheit der nicht leitfähigen Kett- und Schussfäden in der Interdigitalstruktur bestimmt. Durch den Einsatz entsprechend feiner Garne betragen die Abstände der Kammelektroden in der Interdigitalstruktur ca. 200 µm, sodass bereits bei geringer Restfeuchte in der textilen Interdigitalstruktur der zur Wirkstoffabgabe notwendige Redoxprozess und Ionentransport über die zwischen den Kammelektroden angelegte Spannung ausgelöst werden kann. So können beim Einsatz von Polyamid - Monofilamenten eine Basisfeinheit der Polyamidfäden von 22 dtex/f1 bei Schussdichten bis zu 96 cm⁻¹, was einem Fadenabstand von 104 µm entspricht, erzielt werden. Bei den durch Schneiden erzeugten gewebten Interdigitalstrukturen kommt zwischen den Elektrodenfäden zusätzlich ein isolierender Schussfaden zum Einsatz, so dass sich ein Abstand zwischen den wirkstoffbeladenen Arbeitselektrodenfäden und dem Gegenelektrodenfaden von 208 µm (Filamentmitte zu Filamentmitte) ergibt. Berücksichtigt man, dass die Monofilamente einen Radius von 60 µm besitzen, reduziert sich die eigentliche Distanz zwischen den redoxaktiven Elektrodenfäden auf 148 µm.

Durch den Einsatz feiner Multifilamente (PA 34 dtex/f7) als Isolationsschuss kann die Feuchtigkeitsaufnahme zwischen den am Redoxprozess beteiligten Kammelektroden noch begünstigt werden. Werden an Stelle der Monofilamentkettfäden ebenfalls feine Multifilamente (PA 34 dtex/f7) verwendet, kann die Feuchteaufnahme bei Beibehaltung des geringen Elektrodenabstandes von weniger als 150 µm weiter gesteigert werden. Da für textile interaktive TTS keine großflächigen Gewebe zum Einsatz kommen müssen, ist es sinnvoll, die Fertigung von textilen Interdigitalstrukturen auf Bandgewebe zu übertragen. Da bei Bandwebmaschinen der Schuss über einen sogenannten Nadeleintrag erfolgt, wird bei jedem Schusseintrag ein Doppelschuss eingetragen, so dass Schusseinträge von 96 cm⁻¹ unrealistisch sind. Allerdings wird deutlich, dass über die Leinwandbindung im Gewebe die redoxaktiven Fadenelektroden bereits gegeneinander durch die Webkette isoliert sind. Über eine entsprechende Einbindung ist es möglich, auf das Schneiden der Kammstruktur zu verzichten und die elektrischen Anschlüsse webtechnisch zu erzeugen. Die interdigitalen Bandgewebe können in vielfältiger Weise (elektrolumineszierende Gewebe, Sensoren und interaktive TTS) zum Einsatz gelangen. Es kann bei einer webtechnisch erzeugten Interdigitalstruktur auf den isolierenden Schussfaden zwischen den Elektrodenfäden verzichtet werden.

Neben den gewebten Interdigitalstrukturen bieten sich noch weitere Lösungsansätze zur Realisierung eines entsprechenden textilen Gewebes an, wie im Folgenden erläutert wird. Dies sind insbesondere eine Kaschierung dreier textiler Flächen aus dem wirkstoffbeladenen redoxpolymermodifizierten Elektrodenmateriel, einer ionenleitenden textilen Fläche und aus Polyamid, einer Kaschierung von Drehergeweben aus redoxpolymermodifizierten und wirkstoffbeladenen Schussfäden und Polyamid - Schussfäden zusammen mit nichtleitenden Kettfäden mit hoher Feuchteaufnahme und schließlich eine mehrlagige Gewebekonstuktion, die so aufgebaut ist, dass eine Lage aus redoxpolymermodifiziertem, wirkstoffbeladenem Garn und eine Lage aus Polyamid nicht im direkten elektrischen Kontakt stehen. Alle diese textilen Strukturen weisen die Gemeinsamkeit auf, dass bei ihnen der wirkstoffbeladene leitfähige Faden bzw. textile Fläche in einem engen, jedoch nicht direktem elektrischen Kontakt steht.

Der kaschierende Aufbau lässt sich am einfachsten realisieren. Allerdings reduzieren die für die Kaschierung eingesetzten Kleber und Haftvermittler die ionische Leitfähigkeit dieser Anordnung. Für unterschiedliche Wirkstoffapplizierungen können mit dem entsprechenden Wirkstoff beladene textile Flächen präpariert werden, aus denen das eigentliche textile interaktive TTS gefertigt wird.

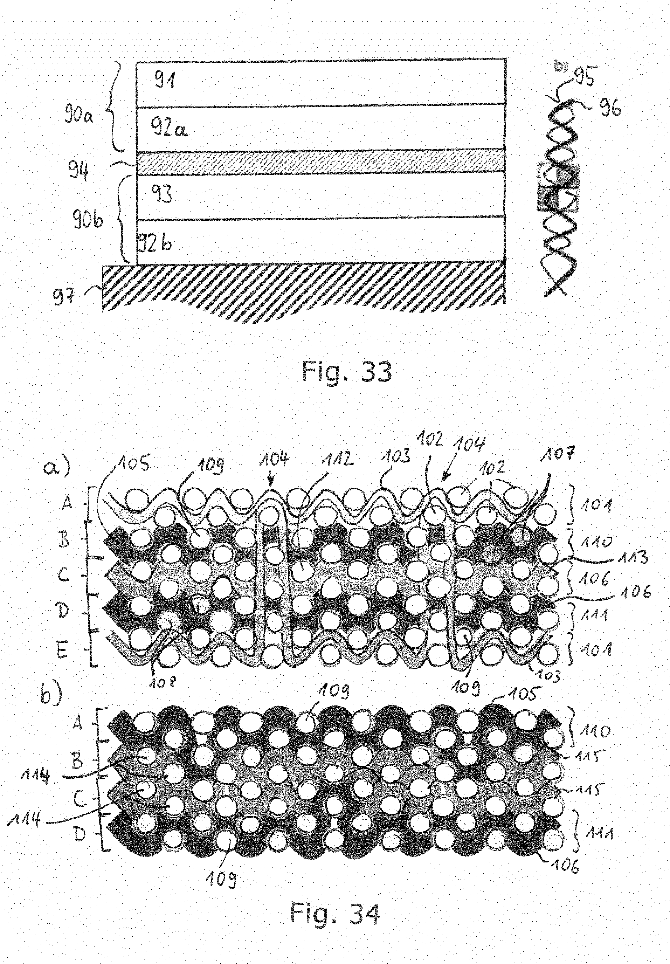

Ein beispielhaftes Drehergewebe ist in Fig. 33 in seinem Aufbau gezeigt. Es ist als ein leinwandgebundenes Drehergewebe mit zwei sich in der Garnfeinheit unterscheidenden Kettfäden ausgebildet, die alternierend in eine Webkette eingezogen werden. Wechseln sich Steher- und Dreherfaden ab, resultiert daraus ein Gewebe, dessen eine Gewebeseite kein Schussmaterial enthält und in dessen zweiter Seite der Kettfaden mit der geringeren Feinheit nicht vorkommt. Auf diese Weise werden die Eigenschaften einer Gewebeseite lediglich durch das Schussmaterial bestimmt, das im Gegensatz zum Kettmaterial ohne großen Aufwand ausgetauscht werden kann. Über das Schussmaterial kann folglich festgelegt werden, welche interaktiven Eigenschaften das Gewebe haben bzw. welcher Wirkstoff abgegeben werden soll. Liegt das wirkstoffbeladene redoypolymermodifizierte ELITEX®-Material als Garn vor, kann die Produktion schnell, von einem interaktiven TTS auf ein anderes ohne Rüstzeiten und Maschinenumstellung umgestellt werden. Allerdings ist auch hier noch eine Kaschierung der beiden textilen Basisflächen (wirkstoffabgebende Fläche und Gegenelektrodenfläche) notwendig.

Fig. 33 zeigt ein diesbezügliches Beispiel. In der Figur ist ein Aufbau eines textilen interaktiven TTS aus zwei Drehergeweben 90a und 90b gezeigt, bei denen ein isolierender Multifilamentkettfaden eine erste und der Schussfaden eine andere Gewebeseite bildet. Diese bestehen im Drehergewebe 90a aus einer nichtleitenden Kette 91 und einem AgCl/Ag-Schuss 92 als Elektrode. In entsprechender Weise weist das Drehergewebe 90b eine nichtleitende Kette 93 und einen redoxpolymermodifizierten und wirkstoffbeladenen Schuss 92 auf. Diese beiden Drehergewebe werden durch ein Hotmelt-Web 94 verbunden. Dies kann beispielsweise ein elektrisch leitfähige Klebematerial sein. Die Drehergewebe selbst werden durch einen zweiten dünneren meist Monofilamentfaden zusammengehalten. Das Teilbild b) zeigt eine schematische Darstellung des Verlaufs der beiden alternierend eingezogenen unterschiedlichen Kettfäden 95 und 96 und ihrer Einbindung im Falle einer Leinwandbindung. In dem hier dargestellten Beispiel liegt der Kettfaden 96 mit der geringeren Feinheit immer über dem Kettfaden 95 mit der größeren Feinheit.

Die Kaschierung kann eingespart werden, wenn es gelingt ein Gewebe zu konzipieren, in dem der wirkstoffbeladene Faden und der Gegenelektrodenfaden jeweils in getrennten sich nicht berührenden Schichten liegen, die jedoch partiell bindungsseitig miteinander verbunden sind. Wird das Garn, dass als Gegenelektrode dient, in der Webkette so eingezogen, dass es nur in einer Gewebelage auftaucht und wird das wirkstoffbeladenen Material im Schuss in die entgegengesetzte Lage eingetragen, kann dies bereits in einem dreilagigen Gewebe realisiert werden.

Interessant sind auch mehrlagige Gewebekonstruktionen, in denen beide Elektrodenmaterialien als Schussmaterial eingetragen sind. Solche Konstruktionen sind in Fig. 34 für ein fünflagiges bzw. vierlagiges Gewebe gezeigt. Bei dem fünflagigen Gewebe sind eingezogene Elektrodenflächen 100 zusätzlich mit jeweils einer Gewebelage 101 nach Außen abgedeckt. Über diese Lage erfolgt der der bindungsseitige Zusammenhalt des fünflagigen Gewebes. Durch einen solchen Aufbau wird ein direkter Hautkontakt der wirkstoffbeladenen textilen Elektrodenfläche vermieden. Das führt einerseits zu einer langsameren Wirkstoffübertragung auf die Haut. Andererseits kann ein direkter Hautkontakt mit den Redoxpolymer-Materialien vermieden werden.

Bei der fünflagigen Konfiguration eine Lage A und eine Lage E durch nicht leitfähige Kettfäden 102 gebildet, zwischen die ein nicht leitfähiger Schussfaden 103 eingewebt ist. Die dadurch gebildeten Gewebelagen 101 stellen nicht leitfähige Deck- oder Abschlusslagen dar. An einzelnen Verbindungsstellen 104 greift der Kettfaden 102 mindestens einer der Lagen 101 durch die gesamte Gewebeanordnung hindurch und hält somit das Gewebe zusammen. Die Lagen B und D werden durch einen Wirkstoffelektroden-Schussfaden 105 und einen Gegenelektroden-Schussfaden 106 gebildet, die zwischen leitfähige Kettfäden 107 und 108 bzw. nicht leitfähige Kettfäden 109 eingewebt sind. Diese bilden eine Wirkstoff-Elektroden-Lage 110 und eine Gegenelektrodenlage 111 aus. Die dazwischen befindliche Lage C besteht aus nicht leitfähigen Kettfäden 112 und einem nicht leitfähigen Schussfaden 113 mit einem hinreichend großen Aufnahmevermögen für Elektrolyte und somit einer hohen Ionenleitfähigkeit.

Bei der vierlagigen Konfiguration in Fig. 34 sind vier Lagen A bis D vorgesehen. Dabei bilden die Lagen A und D jeweils eine Wirkstoff-Elektrodenlage 110 und eine Gegenelektrodenlage 111 mit den entsprechenden Schussfäden 105 und 106 und den Kettfäden 109 aus. Zwischen diesen Elektroden befinden sich in den dazwischen gelegenen Lagen B und C nicht leitfähige Kettfäden 114 und ebenso nicht leitfähige Schussfäden 115 mit einem hinreichend großen Aufnahmevermögen für Elektrolyte und einer entsprechend hohen Ionenleitfähigkeit.

Die vierlagige Konfiguration kann vor allem für Funktionstests bevorzugt werden. Der Zusammenhalt der Gewebekonstruktion wird durch Einbindungen der ersten wirkstoffhaltigen Gewebelage A in die zweite nichtleitende Lage C und Bindungen zwischen den beiden nichtleitenden Gewebelagen B und C sowie Einbindungen der vierten aus Polyamid-Schussfäden bestehenden Lage D in die dritte nichtleitende Lage C gesichert.

Für einen Funktionstest dieses Aufbaus können zunächst Muster gefertigt werden, bei denen die spätere wirkstoffhaltige Gewebelage nur durch Polyamid - Garn gebildet wird. An Hand dieser Muster kann nachgewiesen werden, dass die Lagen A und D tatsächlich nicht leitend verbunden sind. In einem zweiten Test, bei dem gleichzeitig die Verwebbarkeit von mit Redoxpolymeren modifizierten Polyamid-Garnen getestet wird, können vierlagige Gewebe mit leitfähigen Anschlüssen, die über leitfähige Kettfäden realisiert werden, mit einer Polyamid- und einer mit Redoxpolymeren (PAni und PEDOT) modifizierten Garnlage gewebt werden. An beiden leitfähigen Lagen können die Elektroden über die zusätzlich eingebrachten leitfähigen Kettfäden kontaktiert werden.

Die erzeugten Gewebe stellen nach dem Anlöten von Anschlusskabeln an die leitfähigen Kettfäden, deren elektrischer Isolation und Beladung mit einem Wirkstoff bereits ein textiles interaktives TTS dar. Zur Überprüfung kann das Gewebe in eine elektrochemische Messzelle mit einer Gegenelektrode, einer Referenzelektrode und einem 0,1M NaCl-Elektrolyten gebracht werden. Wird nun zunächst nur die redoxpolymermodifizierte Elektrode angeschlossen und ein ausreichend negatives Potential angelegt, entfärbt sich die redoxpolymermodifizierte Gewebelage. Das ist ein Zeichen dafür, dass die Redoxpolymerpräparation auch in der Gewebestruktur reduziert werden kann.

Dies gelingt auch dann, wenn die Messzelle nicht mit Inertgas gespült wird. Überführt man die textile Struktur bei angelegtem Potential direkt in eine Wirkstofflösung z.B. aus Acetylsalicylsäure, färbt sich die Redoxpolymerschicht allmählich wieder, im Fall von PAni dunkelgrün und bei PEDOT entsprechend blau. In der mit Luftsauerstoff gesättigten Lösung werden die zuvor reduzierten Redoxpolymerpräparationen wieder oxidiert. Dabei bilden sich positiv geladenen polaronische und bipolaronische Abschnitte auf der konjugierten Polymerkette aus, deren Ladungen durch die Einlagerung von Anionen an das Polymergerüst ausgeglichen werden müssen. Da die Lösung keine weiteren Anionen als die des Wirkstoffes enthält, kann nur dieser an die Polymerketten ionisch gebunden werden.

Bringt man in die Wirkstofflösung eine externe Gegen- und Referenzelektrode und legt an die redoxpolymermodifizierte Gewebelage ein positive Potential über eine ausreichende Zeit an, gelingt es, die Polymerpräparation fast vollständig zu oxidieren und eine entsprechende Wirkstoffmenge einzulagern. Das Beladen der redoxpolymermodifizierten interaktiven textilen Elektrode gelingt auch ohne die Verwendung einer externen Gegen- und Referenzelektrode durch das direkte Anlegen entsprechender Spannungen zwischen den beiden webtechnisch erzeugten Elektrodenflächen.

Konkret erfolgt das elektrochemische Reduzieren in 0,1 M NaCl bei einer Spannung von etwa -600 mV, einem nachfolgenden Waschen in alkoholischer Acetylsalicylsäure und einem daran anschließenden Oxidieren der Polymerschicht bei einer Spannung von +400 mV in alkoholischer Acetylsalicylsäure. Dabei wird die Polymerschicht mit Acetylsalicylsäure beladen.

Das elektrochemisches Beladen der gewebten textilen redoxpolymermodifizierten Polyamid - Elektrodenschicht mit Wirkstoff kann wie erwähnt durch direktes Anlegen der Spannung an die gewebten Elektrodenschichten erfolgen. Beim elektrochemischen Reduzieren der Redoxpolymerschicht beobachtet man im Laborversuch einen Farbumschlag von dunkelgrün zu hellblau. Das nachfolgende Waschen der reduzierten Schicht mit der Wirkstofflösung führt zu einem Farbwechsel von hellblau nach grün, das danach ausgeführte elektrochemische Oxidieren der Redoxpolymerschicht in der Wirkstofflösung zeigt sich durch eine Farbänderung von grün nach dunkelgrün an. Das abschließende Waschen der Struktur in Kochsalzlösung erzeugt dann keine farbliche Veränderung mehr.

Um sicherzustellen, dass der Wirkstoff ausschließlich im Redoxpolymer gebunden ist, müssen im Gewebe verbliebene Wirkstoffreste durch Waschen in einer 1 M NaCl-Lösung entfernt werden. Ob die Wirkstofflösung vollständig entfernt worden ist, kann dadurch getestet werden, indem auf getrocknete Gewebeproben eine 0,1 M NaCl-Lösung gegeben und nach einigen Minuten ein Teil der Lösung in eine Küvette überführt wird. Ist in dem von der Küvette aufgenommenen UV-VIS-Spektrum kein Wirkstoffspektrum mehr zu erkennen, war die Wäsche ausreichend.

Für den Test einer interaktiven Wirkstoffabgabe durch elektrochemische Reduktion wird dieser Versuch ohne angelegtes Potential mehrfach über eine längere Zeit wiederholt. Schließlich wird eine Spannung von 1,2 V an die wirkstoffbeladenen redoxpolymermodifizierten Polyamid-Elektriode und die Gegenelektrode angelegt. Dabei liegt der negative Pol der Spannungsquelle an die wirkstoffbeladene Schicht für die Dauer von etwa einer Minute an. Dabei entfärbt sich die Gewebelage vollständig und die rückseitige Polyamid-Lage bekommt eine dunkelgraue Färbung.

Die Farbveränderungen weisen darauf hin, dass die Redoxpolymerpräparation reduziert und die Lage der Gegenelektrode oxidiert worden ist und sich mit Silberoxid überzogen hat. In der reduzierten Redoxpolymerschicht sind die Wirkstoffanionen nicht mehr ionisch gebunden. Sie können demnach mit einer 0,1 M NaCl-Lösung, die dem Hautschweiß entspricht, ausgewaschen werden. Gibt man erneut 5 mL 0,1 M NaCl-Lösung in einer Petrischale auf das Gewebe und überführt einen Teil der Lösung in eine Küvette, können die Absorptionsbanden des Wirkstoffes im Spektrum deutlich nachgewiesen werden.

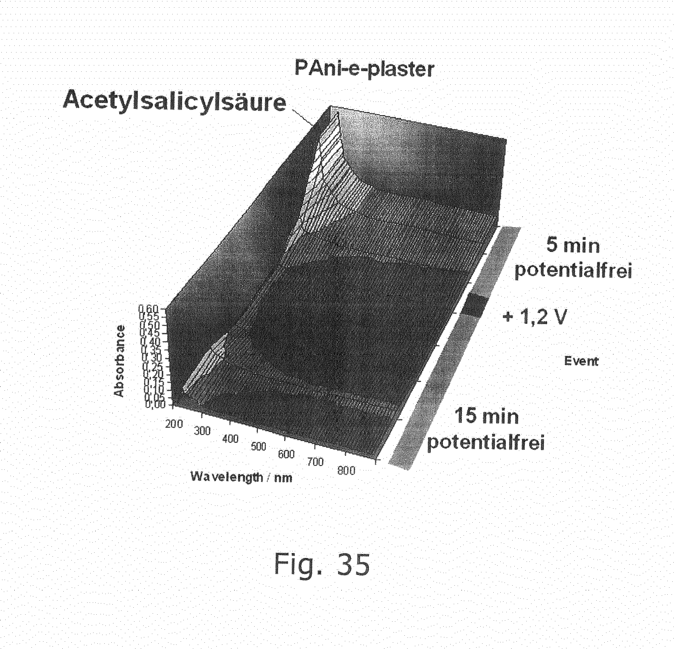

Dass dies tatsächlich der Fall ist, zeigen sie auf diese Weise aufgenommenen und in der 3D-Darstellung in Fig. 35 beispielhaft dargestellten Spektren. Während vor dem Anlegen der Gleichspannung kein Wirkstoff aus dem Gewebe ausgewaschen werden kann, gelingt dies deutlich nach dem Anlegen der Gleichspannung. Um die abgegebene Wirkstoffmenge in erster Näherung quantitativ erfassen zu können, kann die Lösung aus der Küvette bei der nächsten Spülung des Gewebes wieder eingesetzt werden.

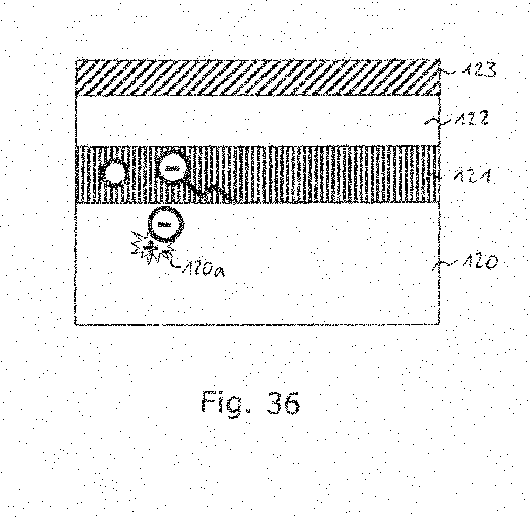

Die Möglichkeit, Ionen austauschende Redoxpolymerschichten für die Wirkstofffreisetzung aus einer mit einem kationischen Wirkstoff beladenen Polymerschicht freizusetzen, ist schematisch in den Figuren 36, 37 und 38 dargestellt. In 36 ist ein dafür geeigneter prinzipieller Schichtaufbau abgebildet. Auch diese Schichtstruktur kann wieder durch eine Interdigitalstruktur realisiert werden. Die Schichtstruktur besteht aus einer Polymermatrix 120 mit darin enthaltenem kationischen Wirkstoff 120a, einem darüber gelagerten leitfähigen Polymer 121, einer diese wiederum bedeckende ionenleitfähige Polymerschicht 122 und einer Ag/AgCl-Schicht 123.

Bei einer Realisierung dieser Schichtstruktur durch eine gewebte Interdigitalstruktur ist eine der textilen Kammelektroden mit einem anionenaustauschenden Redoxpolymer präpariert und die andere mit Ag/AgCl. Eine Seite der Interdigitalstruktur ist mit einem ionenleitfähigen Polymer beschichtet und die andere Seite mit einer kationenwirkstoffhaltigen Polymerschicht präpariert. Dabei entsteht ein textiles TTS, bei dem sich die Haut lediglich in Kontakt mit der wirkstoffhaltigen Polymerschicht und nicht mehr mit den Redoxpolymeren befindet. Auf diese Weise ergibt sich zwischen den galvanisch versilberten Polyamid-Garnen und der Haut kein direkter Kontakt. Dies ist hinsichtlich der Hautfreundlichkeit vorteilhaft.

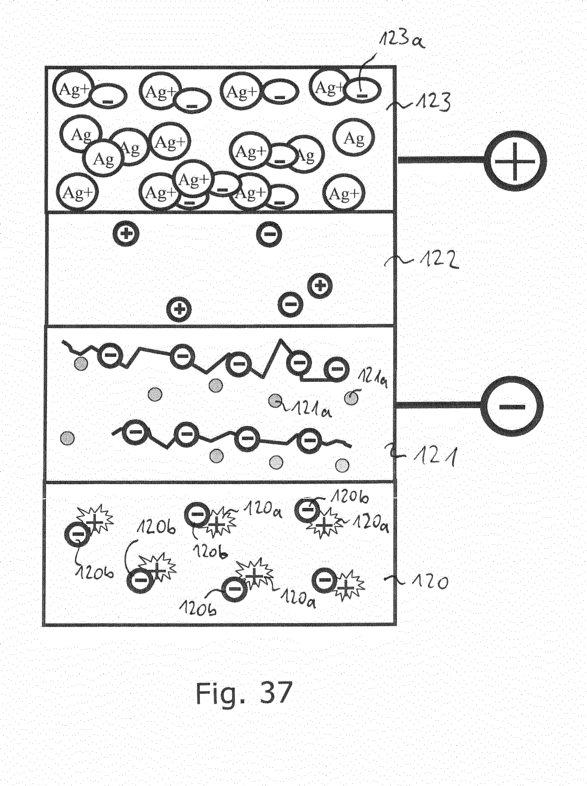

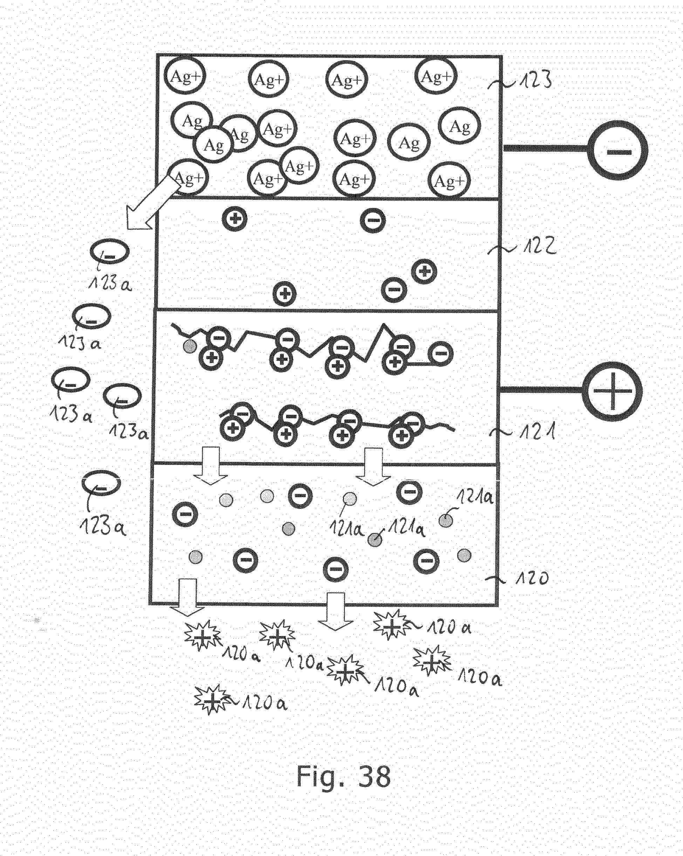

Die Figuren 37 und 38 zeigen grundsätzliche Vorgänge innerhalb des Schichtaufbaus nach Fig. 36 zur Speicherung und Freisetzung des kationischen Wirkstoffes. In Fig. 37 ist der Schichtaufbau so gepolt, dass das Redoxpolymer reduziert ist. In diesem Fall werden negativ geladene Ionen 123a in der Ag/AgCl-Schicht 123 gebunden, während positiv geladene Ionen 121a, d.h. Kationen, in der Redoxpolymerschicht 121 gespeichert werden. In der Polymermatrix 120 ist unter dieser Bedingung der kationische Wirkstoff an innerhalb der Matrix vorhandene unbewegliche negative Ladungen 120b gebunden.

Wird, wie in Fig. 38 dargestellt, die Polung gewechselt, so wird das Redoxpolymer oxidiert. Unter diesen Bedingungen neigt das Redoxpolymer zu einem Kationenaustausch. Das bedeutet, dass aus der Schicht des Redoxpolymers die Kationen 121a freigesetzt werden. Diese wandern in die Polymermatrix 120 aus und verdrängen den dort gespeicherten kationischen Wirkstoff 120a, der somit aus der Polymermatrix austritt und von der Hautoberfläche aufgenommen werden kann.

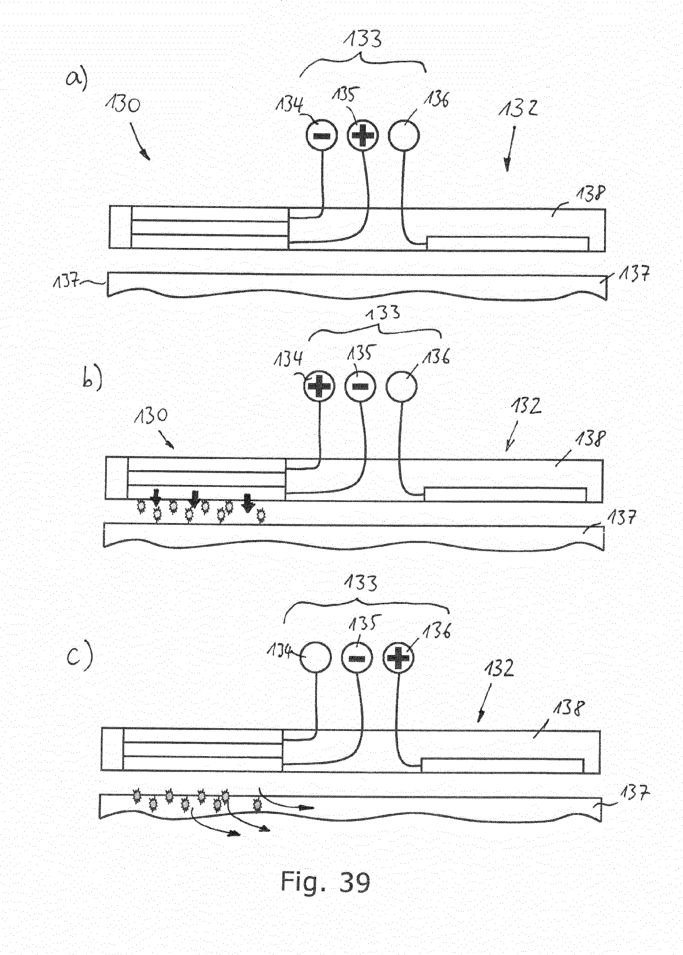

Ein interessanter Aspekt ist die Kopplung der vorhergehend behandelten textilen TTS und der interaktiven Wirkstofffreisetzung über mit Redoxpolymeren modifizierten textilen Polyamid-Strukturen mit einer Iontophorese. Ein Beispiel ist hierzu in Fig. 39 gezeigt. Sowohl bei der Freisetzung des Wirkstoffs aus dem Redoxpolymer als auch bei der Iontophorese kommen ionische Wirkstoffe zum Einsatz. So reicht die Palette bei der Ionthophorese von kationischen Wirkstoffen wie z.B. Procain, Acetylcholin, AlphaChymocutan, Bienengift, Histamin, Hyaluronidase zu anionischen Wirkstoffen wie z.B. Jodat, Ichtyol, Ascorbinat, verschiedene Salicylate und Nicotinat. Letzte sind die vorhergehend als Beispiel genannte Anionen (Acetylsalicylat und Nicotinat), bei denen die interaktive Freisetzung im aus mit Redoxpolymer präparierten Textilien gelungen ist. Eine Kombination beider Methoden bietet sich somit auf der Basis der gleichen Wirkstoffe und wegen der eingesetzten elektrischen Stimulationen an. So sind beispielsweise polyelektrolytische Präparationen zur Verbesserung des elektrischen (richtiger des ionischen) Kontaktes zwischen leitfähigen textilen Elektroden und der Haut möglich, deren Kontaktwiderstände auch bei sehr geringen Präparationsauflagen denen von kommerziellen Gel - Klebeelektroden entsprechen und teilweise deutlich unterschreiten.

Die Kombination dieser Präparationen mit der Iontophorese und der entwickelten neuen Form der interaktiven Wirkstoffabgabe aus redoxpolymermodifizierten leitfähigen textilen Strukturen eröffnen die Möglichkeit der Entwicklung äußerst effektiver textiler transdermaler therapeutischer Systeme, bei denen der Wirkstoff nicht nur zum gewünschten Zeitpunkt appliziert sondern auch gezielt durch die Barriere der Haut gebracht werden kann.

Fig. 39 zeigt eine schematische Darstellung des Aufbaus eines interakiven textilen transdermalen therapeutischen Systems mit gekoppelter Iontophorese in den drei Zuständen des Speichern des Wirkstoffs im oxidierten Redoxpolymer a), der Freisetzung des Wirkstoffes durch Reduktion des Redoxpolymers b) und des iontophoretischen Transportes des Wirkstoffes durch die Haut c).

Die gesamte Anordnung umfasst das vorhergehend erläuterte textile TTS unter Verwendung des Redoxpolymers in einer der vorhergehend erwähnten Ausführungsformen. Diese bildet einen Speicherabschnitt 130. Eine textile Anode ist als ein iontophoretischer Abschnitt 132 ausgeführt. Die Anode ist beispielsweise in Form einer Carbonelektrode realisiert. Zusätzlich ist eine Anschlussanordnung 133 aus drei Anschlüssen vorgesehen, die auf variable Potentiale gelegt werden können. Ein erster Anschluss 134 mündet auf die textile Gegenelektrode des textilen TTS ein, ein zweiter Anschluss 135 ist auf die Redoxpolymerelektrode des TTS gelegt, während ein dritter Anschluss 136 mit der Carbonanode des iontophoretischen Abschnitts 132 verbunden ist. Das textile TTS und die Carbonanode liegen jeweils auf einer äußeren Hautschicht, der Epidermis 137, auf. Das TTS und die Carbonanode sind beide in einen Grundkörper 138 integriert, der als ganzes auf der Hautoberfläche platziert wird.

Die einzelnen Kontakte 134, 135 und 136 können je nach Betriebszustand mit unterschiedlichen Potentialen beaufschlagt werden. Bei dem in Fig. 39a gezeigten Zustand liegt der Anschluss 134 auf negativen Potential und der Anschluss 135 auf positivem Potential. Unter dieser Bedingung wird das vorhergehend beschriebene Speichern des Wirkstoffes im Redoxpolymer der textilen Elektrode ausgeführt. Für das in Fig. 39b gezeigte Freisetzen des Wirkstoffes werden die Vorzeichen der Potentiale an den Anschlüssen 134 und 135 vertauscht. Der Wirkstoff wird nun aus der textilen Elektrode freigesetzt und lagert sich auf der Epidermis 137 ab. In dem in Fig. 39c gezeigten Schritt des Übertragens mittels der Iontophorese wird nun der Anschluss 136 auf ein positives Potential geschaltet. Das elektrische Feld greift nun von der Wirkstoffelektrode durch die obere Epidermis hindurch auf die Carbonanode 132 hindurch und bewirkt einen Transport der Wirkstoffionen in die Hautoberfläche hinein. Dadurch wird der langsame Vorgang einer Wirkstoffverbreitung durch Diffusion zusätzlich beschleunigt und der Wirkstoffeintrag intensiviert. Die oberen Hautschichten nehmen bei der Verschaltung gemäß Teilbild Fig. 93c faktisch die Funktion eines Ionenleiters ein.

Eine derartige, mit einem iontophoretischen Transport gekoppelte Wirkstofffreisetzung ist beispielsweise zweckmäßig bei Behandlungen von Hyperhidrose (starker Schweissneigung der Haut), zur Therapie bei rheumatische Erkrankungen, bei denen der Arzneistoff in Gelenkbereiche eingebracht werden soll, zur Behandlung atropher Hautnarben zum Einschleusen des Wirkstoffes Tretinoin mittels Iontophorese in die Haut. Zur Behandlung der Cellulite kann Androstanolon-haltiges Gel u.a. mittels Iontophorese in das betroffene Unterhautbindegewebe eingeschleust werden. Die genannte Vorrichtung bzw. das Verfahren kann auch zur Diagnose der Erbkrankheit Mukoviszidose verwendet werden. Dabei wird ein Schweißtest in Form einer Pilocarpin-Iontophorese angewendet. Weitere Einsatzgebiete sind die Behandlung der Sklerodermie durch Einschleusen von Hyaluronidase, die Behandlung von Hautpigmentierungen nach Verletzungen durch Ascorbinsäure und das Erweichen von Hautnarben durch iontophoretisch appliziertes Jodat.

Die Effektivität der Anordnung kann dadurch gesteigert werden, indem die Carbonanode 136 durch eine textile Elektrode ersetzt wird. Des weiteren sind Elektrodenpräparationen mit einem Polyelektrolytfilm und/oder einer ionischen Flüssigkeit zur Effektivitätssteigerung der Elektrode 136 sehr vorteilhaft.

Dies bietet die Möglichkeit, eine derartige Elektrode auch zum Ableiten körpereigener bzw. zum Einbringen körperfremder elektrischer Signale zu verwenden. Eine derartige Elektrode ist damit über die reine Iontophorese hinaus im Bereich einer Sensorik bzw. Aktuatorik anwendbar, wobei körpereigene Signale erfasst oder Körperabschnitte stimuliert werden können.

Die Gegenstände der Erfindung wurden anhand von Ausführungsbeispielen dargestellt. Im Rahmen fachmännischen Handelns sind weitere Ausführungsformen möglich. Weitere Ausführungsformen ergeben sich insbesondere aus den Unteransprüchen.

### Bezugszeichenliste

- 1: dreilagige Gewebestruktur
- 2: gewebte Interdigitalstruktur
- 3: metallisierter Faden
- 4: ionenleitendes Polymerfilament
- 5: Redoxpolymer
- 6: Erste Fadenelektrode
- 7: Zweite Fadenelektrode
- 8: Polymermatrix
- 9: Textiler Anodenfaden
- 10: Textiler Katodenfaden
- 11: Nichtleitender Faden
- 12: Multifilament
- 13: Kapillarer Elektrolytfilm
- 14: Elektrisches Feld
- 15: Messzelle
- 16: Abstandstextil
- 17: Flüssigkeitsmenge
- 18: Gleichspannungsquelle
- 19: Leitfähigkeitssensor
- 20: Leitfähigkeitsmessgerät
- 21: Schnittstelle
- 22: PC
- 23: Trenntrafo
- 30: Platin/Silber/Polyamid-Fadenelektrode, positiv
- 31: Platin/Silber/Polyamid-Fadenelektrode, negativ
- 32: Ionenleitfähiges Polymer
- 33: Polymermatrix
- 34: Ag/AgCl- Elektrode
- 35: Ionenleitfähige Schicht
- 36: Redoxpolymermodifizierte Schicht
- 37a: anionischer Wirkstoff
- 37b: positiver polaronischer Polymerabschnitt
- 38: Gegenionen in Lösung
- 40: Küvette
- 40a: Lösung
- 41: Fadenelektrode
- 42: Gegen-Elektrode
- 43: Referenz-Elektrode
- 50: elektrochemische Messzelle
- 51: Stickstoffeinleitung
- 52: Referenzelektrode, gesättigt
- 53: Wirkstoffelektrode, Probe
- 53a: Elektrodenfenster
- 54: spektroskopische Optik
- 55: Magnetrührer
- 60: Garnvorrat
- 61: Polymerdispersion
- 62: Förderrolle
- 63: IR-Strahler
- 64: Thermosolieranlage
- 65: Beschichtetes Garn
- 67: Oberflächenbehandlung
- 68: Metallabscheidung
- 69: Galvanisierung
- 70: Aufbringen Redoxpolymer
- 71: Reduzieren Redoxpolymer
- 72: Waschen
- 73: Oxidieren Redoxpolymer
- 74: Präparierter Garnvorrat
- 75: Fördereinrichtung
- 76: Elektrolytbad
- 76a: Spannungsquelle
- 77: Wirkstoffbad
- 78: Zweites Wirkstoffbad
- 79: Vakuumanlage
- 80: Garnvorrat redoxpolymermodifiziertes Garn
- 85: redoxpolymermodifizierter Elektrodenkamm
- 86: versilbertes Polyamidgarn
- 87: nicht leitfähige Kett- und Schußfäden
- 88: metallisierter Faden
- 90a: erstes Drehergewebe
- 90b: zweites Drehergewebe
- 91: nicht leitfähige Kette
- 92: AgCl/Ag-Schuß
- 93: Nicht leitende Kette
- 94: Hotmelt-Web
- 95: Kettfaden mit größerer Feinheit
- 96: Kettfaden mit geringerer Feinheit
- 100: Elektrodenfläche
- 101: Äußere Gewebelage
- 102: Nicht leitfähiger Kettfaden
- 103: Nicht leitfähiger Schussfaden
- 104: Verbindungsstelle
- 105: Wirkstoffelektroden-Schussfaden
- 106: Gegenelektroden-Schussfaden
- 107: Leitfähiger Kettfaden
- 108: Leitfähiger Kettfaden
- 109: Nicht leitfähiger Kettfaden
- 110: Wirkstoff-Elektrodenlage
- 111: Gegenelektrodenlage
- 112: Nicht leitfähiger Kettfaden
- 113: Nicht leitfähiger Schussfaden
- 114: Leitfähiger Kettfaden
- 115: Leitfähiger Schussfaden
- 120: Polymermatrix
- 120a: kationischer Wirkstoff
- 120b: unbewegliche Anionen
- 121: Redoxpolymerschicht
- 121a: Kation
- 122: Ionenleitfähige Polymerschicht
- 123: Ag/AgCl-Schicht
- 130: Speicherabschnitt
- 132: iontophoretischer Abschnitt mit Carbonanode
- 133: Anschlussanordnung
- 134: Anschluss für textile Gegenelektrode
- 135: Anschluss für Redoxpolymerelektrode
- 136: Anschluss für Carbonanode
- 137: Hautoberfläche
- 138: Grundkörper

## Patentansprüche

1. Vorrichtung zum Ausführen eines Verfahrens zum Speichern und elektrochemischen Freisetzen eines Wirkstoffes in einem transdermalen Wirkstoffreservoir mit einer Verwendung eines Wirkstoffspeichers aus einem zu einem Anionenaustausch neigenden Redoxpolymer und einem anionischen Wirkstoff,
**gekennzeichnet durch**
eine Anordnung zur transdermalen Wirkstoffübertragung in Verbindung mit einer Anordnung zum Ausführen einer Iontophorese, umfassend einen Speicherabschnitt (130) mit einem aus dem Redoxpolymer bestehenden Wirkstoffspeicher und einem von dem Speicherabschnitt entfernt angeordneten iontophoretischen Abschnitt (132) im Kontakt mit einer Epidermis in Verbindung mit einer mit variablen Potentialen beaufschlagbaren Anschlussanordnung (133) aus einem ersten Anschluss (134) zur Kontaktierung der Gegenelektrode des Speicherabschnittes, einem zweiten Anschluss (135) zur Kontaktierung einer mit dem Redoxpolymer beladenen Redoxpolymerelektrode des Speicherabschnittes und einem Anschluss (136) einer Elektrode des iontophoretischen Abschnittes.

2. Vorrichtung zum Ausführen eines Verfahrens zum elektrochemischen Speichern und Freisetzen eines Wirkstoffes in einem transdermalen Wirkstoffreservoir, mit einer Verwendung eines Wirkstoffspeichers aus einem zu einem Kationenaustausch neigenden Redoxpolymer als einem intrinsisch elektrisch leitfähigen Polymer mit inkorporierten unbeweglichen Anionen, insbesondere Toluensulfonat oder einem Polymersulfonat auf einem textilen Träger,
**gekennzeichnet durch**
eine Anordnung zur transdermalen Wirkstoffübertragung in Verbindung mit einer Anordnung zum Ausführen einer Iontophorese, umfassend einen Speicherabschnitt (130) mit einem aus dem Redoxpolymer bestehenden Wirkstoffspeicher und einem von dem Speicherabschnitt entfernt angeordneten iontophoretischen Abschnitt (132) im Kontakt mit einer Epidermis in Verbindung mit einer mit variablen Potentialen beaufschlagbaren Anschlussanordnung (133) aus einem ersten Anschluss (134) zur Kontaktierung der Gegenelektrode des Speicherabschnittes, einem zweiten Anschluss (135) zur Kontaktierung einer mit dem Redoxpolymer beladenen Redoxpolymerelektrode des Speicherabschnittes und einem Anschluss (136) einer Elektrode des iontophoretischen Abschnittes.

3. Vorrichtung zum Ausführen eines Verfahrens zum elektrochemischen Speichern und Freisetzen eines Wirkstoffes in einem transdermalen Wirkstoffreservoir, mit einer Verwendung eines Wirkstoffspeichers aus einem zu einem Kationenaustausch neigenden Redoxpolymer (121) in Kontakt mit einer mit einem kationischen Wirkstoff beladenen Reservoir-Polymerschicht (120) auf einem textilen Träger,
**gekennzeichnet durch**
eine Anordnung zur transdermalen Wirkstoffübertragung in Verbindung mit einer Anordnung zum Ausführen einer Iontophorese, umfassend einen Speicherabschnitt (130) mit einem aus dem Redoxpolymer bestehenden Wirkstoffspeicher und einem von dem Speicherabschnitt entfernt angeordneten iontophoretischen Abschnitt (132) im Kontakt mit einer Epidermis in Verbindung mit einer mit variablen Potentialen beaufschlagbaren Anschlussanordnung (133) aus einem ersten Anschluss (134) zur Kontaktierung der Gegenelektrode des Speicherabschnittes, einem zweiten Anschluss (135) zur Kontaktierung einer mit dem Redoxpolymer beladenen Redoxpolymerelektrode des Speicherabschnittes und einem Anschluss (136) einer Elektrode des iontophoretischen Abschnittes.

4. Verwendung einer Vorrichtung nach einem der Ansprüche 1-3 in Form einer aktuatorischen und/oder sensorischen textilen Elektrode zur Übertragung körpereigener und/oder körperfremder elektrischer Signale.
